# EUROPÄISCHE PATENTSCHRIFT

(11) **EP 2 179 749 B1**
(45) Veröffentlichungstag und Bekanntmachung des Hinweises auf die Patenterteilung: **08.08.2012**
(21) Anmeldenummer: 08018548.1
(22) Anmeldetag: 23.10.2008
(51) Int. Cl.: A61L 15/22, C08G 18/64

(54) **Polyurethangelschäume**
Polyurethane gel foams
Mousses de gel de polyuréthane

(43) Veröffentlichungstag der Anmeldung: 28.04.2010
(73) Patentinhaber: Paul Hartmann AG, 89522 Heidenheim (DE)
(72) Erfinder: Eckstein, Axel, Dr., 89522 Heidenheim (DE); Schneider, Christian, 74363 Güglingen (DE)

(56) Entgegenhaltungen:
- EP-A- 0 169 580
- WO-A-00/47241
- US-A- 5 914 125

## Beschreibung

Die vorliegende Erfindung betrifft Polyurethangelschäume und deren Verwendung in der modernen Wundbehandlung, Insbesondere betrifft die vorliegende Erfindung Wundauflagen umfassend Polyurethangelschäume zur Behandlung von mittel bis stark exsudierenden Wunden.

Polyurethangelschäume sind seit einiger Zeit bekannt. So werden mit der internationalen Anmeldung WO 88/01878 A1 selbstklebende Polyurethanschäume oder Polyurethangelschäume beschrieben. Diese Polyurethangelschäume werden als Wundkontaktschicht in Wundauflagen verwendet.

Weiterhin werden mit den internationalen Anmeldungen WO 92/17518 A1 und WO 94107935 A1 hydrophile Schäume aus einem Polyurethangel beschrieben. Diese Schäume werden hergestellt aus mindestens einem Polyetherpolyol, mindestens einem Diisocyanat, Wasser, Beschleuniger und einem oder mehreren Superabsorbern, wobei die Bestandteile vermischt und gegebenenfalls unter Zuführung von Gasen zu Schäumen aufgeschlagen werden.

Zudem wird mit der internationalen Anmeldung WO 97143328 A1 ein selbstklebendes Polyurethangel oder Polyurethanschaumgel beschrieben, das aus einem aliphatischen Diisocyanat, einer Polyolkomponente, einem Stabilisator und Reaktionsbeschleunigern hergestellt wird. Diese Polyurethanschaumgele weisen keinen Anteil an Polysachariden auf.

Mit US 5,833,665 A ist eine Wundauflage zum Fixieren eines Katheters bekannt. Die Wundauflage umfasst ein ein Medikament freisetzendes Wundkissen, das aus einem Isocyanat-Prepolymer und einem Biopolymer hergestellt wird. Das Biopolymer kann u.a. Pektin, Gelatine oder Algin sein.

Darüber hinaus wird mit der internationalen Patentanmeldung WO 00/47241 A1 ein Polyurethanschaum zur Verwendung als Wundkontaktschicht beschrieben, der aus einer Isocyanatkomponente oder einem Isocyanatprepolymer, Wasser und Alginsäure oder einem Salz hiervon hergestellt wird. Gegebenenfalls kann dem Reaktionsgemisch ein einwertiger C₁-C₃-Alkohol zugesetzt werden. Dieser Polyurethanschaum weist durch die inkorporierte Alginsäure eine verbesserte Absorptionsfähigkeit auf.

Mit der WO 01/62818 A1 wird vorgeschlagen, zur Herstellung eines Polyurethan-Haftklebstoffs zwei Polyole oder anderweitige mit NCO-Gruppen reagierende Materialien mit Isocyanaten umzusetzen, wobei sich die mit den Isocyanaten reagierenden Komponenten dahingehend voneinander unterscheiden, dass die erste Polyolkomponente eine molare Masse größer 2000 und die zweite Polyolkomponente eine molare Masse kleiner 2000 aufweist.

Außerdem wird mit der europäischen Patentanmeldung EP 1923077 A1 eine mehrschichtige Wundauflage beschrieben, die eine Wundkontaktschicht aus einem hydrophilen, selbsthaftenden Polyurethan-Elastomer aufweist. Das Elastomer wird dabei aus einem Diisocyanat-Prepolymer und einer polymeren Polyhydroxykomponente hergestellt.

Insgesamt kann hier festgestellt werden, dass die vorgeschlagenen Polyurethangelschäume zumindest teilweise eine zu niedrige Durchlässigkeit für Wundexsudat aufweisen.

Ausgehend vom Stand der Technik ist es daher eine Aufgabe der vorliegenden Erfindung alternative und verbesserte Polyurethangelschäume und damit hergestellte Wundauflagen zur Behandlung von sezemierenden Wunden bereitzustellen. Die Polyurethangelschäume sollen dabei insbesondere zum Einsatz in der modernen Wundversorgung als Wundkontaktschicht geeignet sein. Eine mit diesem Polyurethangelschaum hergestellte Wundauflage soll zudem nicht wundverklebende Eigenschaften besitzen sowie gleichzeitig die Mazeration der die Wunde umgebenden Haut verhindern.

Überraschenderweise wird diese Aufgabe durch einen Polyurethangelschaum gemäß Anspruch 1 gelöst. Dabei wird ein erfindungsgemäßer Polyurethangelschaum zur Verwendung als Wundkontaktschicht aus mindestens folgenden zur Reaktion gebrachten Komponenten erhalten:
a) eine Isocyanatkomponente A mit einer Funktionalität f von f_{A} ≤ 3,
b) eine polymere Polyolkomponente B mit einer Funktionalität f von f_{B} ≤ 6 und
c) ein Polysaccharid C, das mindestens eine Uronsäure oder ein Salz hiervon umfasst,
wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht.

Unabhängig hiervon ist auch eine Wundauflage umfassend einen Polyurethangelschaum, insbesondere als Wundkontaktschicht, Gegenstand der vorliegenden Erfindung. Dieser Polyurethangelschaum wird aus mindestens folgenden zur Reaktion gebrachten Komponenten erhalten:
a) eine Isocyanatkomponente A mit einer Funktionalität f von f_{A} ≤ 3,
b) eine polymere Polyolkomponente B mit einer Funktionalität f von f_{B} ≤ 6 und
c) ein Polysaccharid C, das mindestens eine Uronsäure oder ein Salz hiervon umfasst,
wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht.

Gemäß einer weiteren Ausführung der vorliegenden Erfindung kann dabei auch vorgesehen sein, dass das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 3 bis 1 : 30, insbesondere 1 : 4 bis 1 : 30, insbesondere 1 : 4 bis 1:20 und ganz besonders bevorzugt 1 : 5 bis 1 : 20 entspricht.

Hervorzuheben ist hierbei, dass sowohl die polymere Polyolkomponente B als auch das Polysacharid C als Reaktionspartner mit der Isocyanatkomponente A reagieren. Hierbei kann das Polysacharid C mit der Isocyanatkomponente A einerseits mittels vorhandener Hydroxylgruppen unter Ausbildung einer Urethanbindung reagieren und andererseits mittels vorhandener Carboxygruppe der Uronsäure unter Ausbildung einer Amidbindung reagieren. Diese Amidbindung erfolgt unter gleichzeitiger Freisetzung von gasförmigem Kohlenstoffdioxid, wodurch der Polyurethangelschaum ohne Einbringen von zusätzlichen Gasen erhalten wird.

Dieser somit erhaltene Polyurethangelschaum ist besonders hautfreundlich und ruft insbesondere bei direktem Körperkontakt keine Irritationen auf einer Wunde oder der die Wunde umgebenden Haut hervor. Durch die Verwendung der Polysaccharide mit mindestens einer Uronsäure kann ein Polyurethangelschaum mit einer neuartigen Polymerstruktur bereitgestellt werden, der eine verbesserte Durchtrittskinetik für Wundexsudat aufweist. Ein besonderer Vorteil der diesen Polyurethangelschaum umfassenden Wundauflage besteht darin, dass durch die Verwendung des Polyurethangelschaums als Wundkontaktschicht einerseits eine Abstandsschicht zwischen einer absorbierenden Schicht und einer Wunde geschaffen wird und somit auch ansonsten zur Wundverklebung neigende Materialien als absorbierende Schicht verwendet werden können, und andererseits durch den Polyurethangelschaum eine nicht wundverklebende Wundkontaktschicht geschaffen wird, die durch ihren Schaumcharakter einen verbesserten Transport von Wundflüssigkeit von der Wunde zu einer absorbierenden Schicht erlaubt. Darüber hinaus hat sich gezeigt, dass diese Wundauflage die Mazeration, das heißt, die Aufweichung und damit verbundene Schädigung der die Wunde umgebenden Haut, verhindert oder zumindest einschränkt. Somit kann eine Wundauflage bereitgestellt werden, die die. Wunde umgebende Haut schont und die im hohen Maße die Wundheilung fördert.

Insbesondere wird dabei zum Erhalt erfindungsgemäßer Polyurethangelschäume neben den genannten Komponenten bis auf den in den Komponenten enthaltenen Restgehalten kein Wasser zur Reaktion gebracht. Damit ist auch ein Polyurethangelschaum oder eine diesen Polyurethangelschaum umfassende Wundauflage Gegenstand der Erfindung, der durch die folgenden zur Reaktion gebrachten Komponenten erhalten wird:
a) eine Isocyanatkomponente A mit einer Funktionalität f von f_{A} ≤ 3,
b) eine polymere Polyolkomponente B mit einer Funktionalität f von f_{B} ≤ 6 und
c) ein Polysaccharid C, das mindestens eine Uronsäure oder ein Salz hiervon umfasst,
wobei die Reaktion ohne Zusatz an Wasser erfolgt und wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht.

Somit ist auch ein wasserfreier Polyurethangelschaum Gegenstand der vorliegenden Erfindung. Hierbei soll im Zusammenhang mit der vorliegenden Erfindung unter einem wasserfreien Polyurethangelschaum oder jeder wasserfreien Verbindung oder Komponente ein Polyurethangelschaum, eine Verbindung oder Komponente verstanden sein, der/die weniger als 4 Gew.-% Wasser bezogen auf das Gewicht des Polyurethangelschaums oder der jeweiligen Verbindung oder Komponente enthält. Insbesondere enthält ein solcher Polyurethangelschaum oder eine solche Verbindung oder Komponente weniger als 2 Gew.-% Wasser, insbesondere weniger als 1 Gew.-% und ganz besonders bevorzugt weniger als 0,5 Gew.-% Wasser.

Zusätzlich oder alternativ kann gemäß einer weiteren Ausführung der vorliegenden Erfindung das Verhältnis der Anzahl der Isocyanatgruppen der Isocyanatkomponente A zur Gesamtzahl der Carboxygruppen und Carboxylatgruppen in dem Polysaccharid C dem Verhältnis 1 : 0,5 bis 1 : 10 entsprechen. Insbesondere kann hierbei ein Verhältnis der Anzahl der Isocyanatgruppen der Isocyanatkomponente A zur Gesamtzahl der Carboxygruppen und Carboxylatgruppen in dem Polysaccharid C von 1 : 1 bis 1 : 10, besonders bevorzugt 1 : 2 bis 1 : 10 und ganz besonders bevorzugt von 1 : 3 bis 1:10 eingestellt werden. Somit kann ein Polyurethanschaum bereitgestellt werden, der eine besonders gleichmäßige Schaumstruktur aufweist.

Gemäß der vorliegenden Erfindung werden erfindungsgemäße Polyurethangelschäume erhalten, wenn Isocyantkomponenten A eingesetzt werden, die eine Funktionalität f_{A} mit f_{A} ≤ 3 aufweisen. Hierunter sind gemäß einem ersten Aspekt solche Isocyanatkomponenten zu verstehen, die in einem idealisierten Formelbild höchstens drei Isocyanatgruppen aufweisen. Diese Anzahl an reaktiven Gruppen ist gemäß der vorliegenden Erfindung zur Bestimmung der einzusetzenden Verhältnisse der Komponenten einzusetzen. Die wirklich vorliegende mittlere Funktionalität f_{A} einer eingesetzten Isocyanatkomponente A kann von dieser Funktionalität f_{A} abweichen.

Weiterhin sind gemäß einem weiteren Aspekt der vorliegenden Erfindung unter einer Isocyanatkomponente A solche Isocyanate zu verstehen, die aliphatischen, alicyclischen, heterocyclischen, aromatischen, heteroaromatischen Ursprungs sind und eine Funktionalität f von f_{A} ≤ 3 aufweisen, d.h. idealisiert höchstens 3 Isocyanatgruppen aufweisen. Gemäß einem weiteren Aspekt sind hierunter auch solche Isocyanate zu verstehen, die als Isocyanat-Prepolymere bezeichnet werden und aus einem aliphatischen, alicyclischen, heterocyclischen, aromatischen oder heteroaromatischen Di- oder Polyisocyanat und einem Polyol hergestellt sind und die eine Funktionalität f von f_{A} ≤ 3 aufweisen.

Gemäß einer bevorzugten Ausführung der vorliegenden Erfindung können als Isocyanatkomponente A insbesondere solche Isocyanate, insbesondere aliphatische oder alicyclische Isocyanate, eingesetzt werden, die eine Funktionalität f von 1 s f_{A} ≤ 3 aufweisen. Besonders bevorzugt sind Isocyanate, die eine Funktionalität f von 2 ≤ f_{A} ≤ 3 aufweisen.

Um Polyurethangelschäume ausreichender Stabilität zu erzeugen, können gemäß der vorliegenden Erfindung aliphatische oder alicyclische Di- oder Polyisocyanate beziehungsweise Di- oder Polyisocyanate mit nicht aromatisch gebundenen Isocyanatgruppen verwendet werden. Überraschend wurde gefunden, dass aliphatische oder alicyclische Di- oder Polyisocyanate geeignet sind, um auch das sonstige gewünschte Eigenschaftsprofil der Polyurethangelschäume entsprechend der Aufgabe der Erfindung zu erzeugen. Besonders hervorzuheben ist, dass sich die oberflächenspezifische Selektivität der Haftklebeeigenschaften durch Verwendung von aliphatischen oder alicyclischen Di- oder Polyisocyanaten besonders gut einstellen lässt.

Beispiele geeigneter, erfindungsgemäßer Di- oder Polyisocyanate sind als aromatische Diisocyanate MDI (Diphenylmethandiisocyanat), TDI (Toluylendiisocyanat), XDI (XylolDiisocyanat), NDI (Naphthalin-Diisocyanat) oder Phenylendiisocyanat. Insbesondere sind jedoch als aliphatische oder alicyclische Di- oder Polyisocyanate Dicyclohexylmethandiisocyanat, Butan-1,4-diisocyanat, Tetramethoxybutan-1,4-diisocyanat, Hexan-1,6-diisocyanat, Ethylendiisocyanat, 2,2,4-Trimethylhexamethylendiisocyanat, Ethylethylendiisocyanat, Dicyclohexylmethan-diisocyanat, 1,4-Diisocyanatocyclohexan, 1,3-Diisocyanatocyclohexan, 1,2-Diisocyanatocyclohexan, 1,3-Diisocyanatocyclopentan, 1,2-Diisocyanatocyclopentan, 1,2-Diisocyanatocyclobutan, 1-Isocyanatomethyl-3-isocyanato-1,5,5-trimethylcyclohexan (Isophorondiisocyanat, IPDI), 1-Methyl-2,4-diisocyanatocyclohexan, 1,6-Diisocyanato-2,2,4-trimethylhexan, 1,8-Diis-ocyanato-2,4,4-trimethylhexan, 5-Isocyanato-1-(2-isocyanatoeth-1-yl)-1,3,3-trimethylcyclohexan, 5-Isocyanato-1-(3-isocyanatoprop-1-yl)-1,3,3-trimethylcyclohexan, 5-Isocyanato-1-(4-isocyanatobut-1-yl)-1,3,3-trimethylcyclohexan, 1-Isocyanato-2-(3-isocyanatoprop-1-yl)-cyclohexan, 1-Isocyanato-2-(2-isocyanatoeth-1-yl)-cyclohexan, 2-Heptyl-3,4-bis(9-isocyanatononyl)-1-pentylcyclohexan oder Norbonandiisocyanatomethyl einzusetzen. Es können jedoch auch chlorierte, bromierte, schwefel- oder phosphorhaltige aliphatische oder alicyclische Di- oder Polyisocyanate, sowie Derivate der aufgeführten Diisocyanate, insbesondere dimerisierte oder trimerisierte Typen eingesetzt werden. Insbesondere können erfindungsgemäß hierbei Uretdione oder Isocyanurate aliphatischer oder alicyclischer Di- oder Polyisocyanate verwendet werden.

Gemäß einer weiteren Ausführung der Erfindung sind hierbei Polyurethangelschäume, die aus aliphatischen oder alicyclische Di- oder Polyisocyanaten oder Mischungen hiervon hergestellt werden, bevorzugt. Insbesondere sind dabei linear aliphatische oder alicyclische Diisocyanate bevorzugt, wobei weiterhin fünf- oder sechsgliedrige alicyclische Diisocyanate bevorzugt sind. In einer besonders bevorzugten Ausführungsform wird isophorondiisocyanat als sechsgliedriges alicyclisches Diisocyanat verwendet, welches eine sehr gute Durchtrittskinetik für Wundexsudat zulässt.

Weiterhin bevorzugt sind als Isocyanatkomponente Prepolymere aus aliphatischen oder alicyclischen Di- oder Polyisocyanaten und Di- oder Polyolen, weiterhin bevorzugt Prepolymere aus alicyclischen Diisocyanaten, wobei insbesondere als Polyole Polyetherpolyole oder Polyesterpolyole Verwendung finden.

Solche Isocyanat-Prepolymere werden durch Umsetzung von höhermolekularen Polyhydroxylverbindungen (Polyole) wie z. B. Polyether- oder Polyester-Polyolen mit überschüssigen Mengen Di- oder Polyisocyanat hergestellt. Dabei ist es von Vorteil, wenn die Isocyanatgruppen unterschiedliche Reaktivitäten aufweisen. Durch diese Eigenschaft wird der unerwünschte Monomerengehalt zurückgedrängt. Die Produkte weisen zudem niedrige Viskositäten und bessere technische Verarbeitbarkeit auf. Beispielsweise besitzt Isophorondiisocyanat (3-Isocyanatomethyl-3, 5,5-trimethylcyclohexylisocyanat, IPDI) zwei solche unterschiedlich (selektiv) reaktive Isocyanatgruppen.

Damit sind gemäß einer weiteren vorteilhaften Ausführung insbesondere solche Polyurethangelschäume bevorzugt, die erhalten werden, wenn als Isocyanatkomponente A Isophorondiisocyanat, ein Isophorondiisocyanat-Prepolymer oder eine Mischungen hiervon eingesetzt wird, wobei das Isophorondiisocyanat-Prepolymer erhältlich ist aus Isophorondiisocyanat und einem Polyetherpolyol D mit einer Funktionalität f von f_{D} ≤ 3.

Ganz besonders bevorzugt sind Polyurethangelschäume, die erhalten werden, wenn als Isocyanatkomponente A ein Isophorondiisocyanat-Prepolymer eingesetzt wird, das erhältlich ist aus Isophorondiisocyanat und einem Polyetherpolyol D, wobei das Polyetherpolyol D ausgewählt wird aus der Gruppe der Polyethylenglykole, Polypropylenglykole, Polyethylen-Polypropylenglykole oder Mischungen hiervon, und wobei das Polyetherpolyol D insbesondere eine mittlere zahlengemittelte molare Masse Mₙ von mindestens Mₙ (D) = 2000 g/ mol und höchstens Mₙ (D) =10.000 g/ mol aufweist.

Gemäß der vorliegenden Erfindung kann damit als Isocyanatkomponente A insbesondere ein Isophorondiisocyanat-Prepolymer gemäß Formel (I) zur Reaktion gebracht werden.

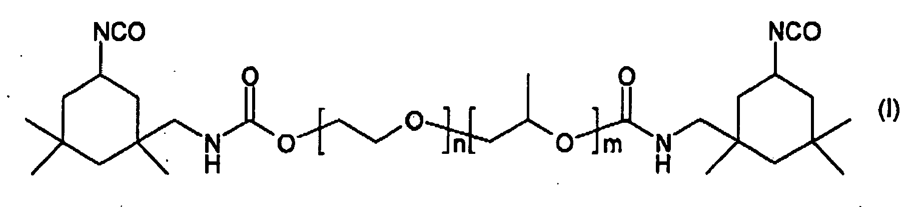

Hierbei bedeuten:
n, m unabhängig voneinander: n, m = 0 bis 120, wobei gleichzeitig n + m ≠ 0 gilt.

Insbesondere werden gemäß der vorliegenden Erfindung solche Isocyanat-Prepolymere gemäß Formel (I) bevorzugt, für die n = 0 und m =1 bis 120 gilt. Ganz besonders bevorzug werden solche Prepolymere eingesetzt, für die n = 0 und m = 30 bis 120, besonders bevorzugt n = 0 und m = 30 bis 90 und ganz besonders bevorzugt n = 0 und m = 40 bis 80 gilt.

Gemäß einer weiteren Ausführung der vorliegenden Erfindung können jedoch auch solche Prepolymere gemäß Formel (I) verwendet werden, für die n =1 bis 120 und m = 0 gilt. Besonders bevorzug werden dabei solche Prepolymere eingesetzt, für die n = 30 bis 120 und m = 0, besonders bevorzugt n = 30 bis 90 und m = 0 und ganz besonders bevorzugt n = 40 bis 80 und m = 0 gilt.

In der Regel weisen die im Rahmen der vorliegenden Erfindung einsetzbaren Isocyanst-Prepolymere, insbesondere Diisocyanat-Präpolymer, eine mittlere zahlengemittelte molare Masse von etwa 500 g/ mol bis etwa 15.000 g/ mol, vorzugsweise etwa 500 g/ mol bis etwa 10.000 g/ mol, besonderes bevorzugt etwa 1.000 g/ mol bis etwa 10.000 g/ mol, besonders bevorzugt etwa 2.000 g/ mol bis etwa 10.000 g / mol und ganz besonders bevorzugt 3.000 g / mol bis etwa 10.000 g / mol auf.

Gemäß der vorliegenden Erfindung werden erfindungsgemäße Polyurethangelschäume erhalten, wenn als polymere Polyolkomponente B solche Polyolkomponenten eingesetzt werden, die eine Funktionalität f von f_{B} ≤ 6 aufweisen. Hierunter sind analog der Isocyanatkomponente A gemäß einem ersten Aspekt solche Polyolkomponenten zu verstehen, die in einem idealisieren Formelbild höchstens sechs Hydroxygruppen aufweisen. Diese Anzahl an reaktiven Gruppen ist gemäß der vorliegenden Erfindung zur Bestimmung der einzusetzenden Verhältnisse der Komponenten einzusetzen. Die wirklich vorliegende mittlere Funktionalität f̅ _{B} einer eingesetzten Polyolkomponente kann von dieser. Funktionalität f_{B} abweichen.

Gemäß einer bevorzugten Ausführung der vorliegenden Erfindung können als Polyolkomponente B insbesondere solche Polyole, insbesondere Polyetherpolyole, eingesetzt werden, die eine Funktionalität f von 3 ≤ f_{B} ≤ 6 aufweisen. Besonders bevorzugt sind Polyetherpolyole, die eine Funktionalität f von 3 ≤ f_{B} ≤ 5 aufweisen. Ganz besonders bevorzugt sind Polyetherpolyole, die eine Funktionalität f von 4 ≤ f_{B} ≤ 5 aufweisen.

Beispiele geeigneter erfindungsgemäßer Diole oder Polyole sind Oxyalkyl-Polymere, vorzugsweise 2, 3, 4, 5 oder 6 Hydroxylgruppen aufweisende Polyetherpolyole mit OH-Zahlen von 20 bis 112 und einem Ethylenoxid-Gehalt von ≥ 10 Gew.-%, vorzugsweise 10 bis 40 Gew.-%, besonders bevorzugt 10 bis 20 Gew.-%, Polyacrylpolyole, Polyesterpolyole, Polyolefinpolyole, Polythiol-Polyole, Polyamin-Verbindungen. Die Glasübergangstemperaturen sollten hierbei möglichst tief liegen, also unter etwa 20 °C, vorzugsweise unter etwa 0 °C, besonders bevorzugt unter etwa -10 °C.

Polyetherpolyole mit mittleren zahlengemittelten molaren Massen Mₙ (B) zwischen 600 und 12.000 g/ mol sind bevorzugt und können nach bekannten Verfahren bspw. durch Umsetzung der Starterverbindungen mit einem reaktiven H-Atom mit Alkylenoxiden (beispielsweise Ethylen-, und/oder Propylenoxid, vorzugsweise Propylenoxid, Butylenoxid, Styroloxid, Tetrahydrofuran oder Epichlorhydrin oder Gemischen aus zwei oder mehr davon) erhalten werden. Ebenfalls sind Tetramethylenetherglykole einsetzbar. Ebenfalls möglich sind weitere Modifikationen z.B. mit Monoethylenglycol (MEG), Dipropylenglykol (DPG), Trimethylolpropan (TMP). Bevorzugt für den Einsatz in der Medizin werden heute aliphatische Polyetherpolyole eingesetzt.

Geeignete Startverbindungen sind beispielsweise Wasser, Ethylenglykol, Propylenglykol-1,2 oder-1,3, Butylenglykol-1,4 oder-1,3, Hexandiol-1,6, Octandiol-1,8, Pentandiol-1,5, Heptandiol-1,7, und deren höhere Homologe, Neopentylglykol, 1,4-Hydroxymethylcyclohexan, 2-Methyl-1,3-propandiol, Glyzerin, Trimethylolpropan, 2,2-(bis-4,4'-hydroxyphenyl)-Propan, Trimethylolpropan, Glyzerin oder Pentaerythrit, Hexantriol-1,2,6, Butantriol-1,2,4 Trimethylolethan, Mannitol, Sorbitol, Methylglykoside, Zucker, Phenol, Isononylphenol, Resorcin, Hydrochinon, 1,2,2- oder 1,1,2-Tris-(hydroxyphenyl)-ethan, Ammoniak, Methylamin, Ethylendiamin, Tetra- oder Hexamethylenamin, Triethanolamin, Anilin, Phenylendiamin, 2,4-und 2,6-Diaminotoluol und Polyphenylpolymethylenpolyamine, wie sie sich durch Anilin-Formaldehydkondensation erhalten lassen, oder Gemische aus den vorstehenden Startverbindungen davon.

Insbesondere sind zur Herstellung erfindungsgemäßar Polyurethangelschäume als Polyolkomponente B Polyetherpolyole zu verwenden. Besonders bevorzugt sind dabei Polyetherpolyol mit einem mittleren zahlengemittelten molaren Masse Mₙ (B) von 600 bis 12.000 g/ mol einzusetzen. Insbesondere können jedoch auch Polyetherpolyole eingesetzt werden, die eine mittlere zahlengemittelte molare Masse von Mₙ (B) von 600 bis 10.000 g/ mol, insbesondere von 1.000 bis 10.000 g/ mol, und ganz besonders bevorzugt von 2.000 bis 10.000 g/ mol haben. Als weiterhin vorteilhaft hat sich gezeigt, wenn Polyetherpolyole eingesetzt werden, die eine mittlere zahlengemittelte molare Masse Mₙ (B) von 3.000 bis 10.000 g/ mol aufweisen.

Ebenfalls als Diol- oder Polyolkomponente geeignet sind OH-Gruppen tragende Polyacrylate. Diese werden beispielsweise erhalten durch die Polymerisation von ethylenisch ungesättigten Monomeren, die eine OH-Gruppe tragen. Solche Monomeren sind beispielsweise erhältlich durch die Veresterung von ethylenisch ungesättigten Carbonsäuren und difunktionellen Alkoholen, wobei der Alkohol in der Regel in einem leichten Überschuss vorliegt. Solche ungesättigte Carbonsäuren sind beispielsweise Acrylsäure, Methacrylsäure, Crotonsäure oder Maleinsäure. Entsprechende OH-Gruppen tragende Ester sind beispielsweise 2-Hydroxyethylacrylat, 2-Hydroxyethylmethacrylat, 2-Hydroxy-propylacrylat, 2-Hydroxypropylmethacrylat, 3-Hydroxypropylacrylat oder 3-Hydroxypropyl-methacrylat oder Gemische aus zwei oder mehreren davon.

Ebenfalls als Diol- oder Polyolkomponente geeignet sind Polyesterpolyole, insbesondere mit einer mittleren zahlengemittelten molaren Massen von etwa 200 bis etwa 10.000 g/ mol. So können beispielsweise Polyesterpolyole verwendet werden, die durch Umsetzung von niedermolekularen Alkoholen, insbesondere von Ethylenglykol, Diethylenglykol, Neopentylglykol, Hexandiol, Butandiol, Propylenglykol, Glyzerin oder Trimethylolpropan mit Caprolacton entstehen. Ebenfalls als polyfunktionelle Alkohole zur Herstellung von Polyesterpolyolen geeignet sind 1,4-Hydroxy-methylcyclohexan,2-Methyl-1,3-propandiol, Butantriol-1,2,4, Triethylenglykol, Tetraethylenglykol, Polyethylenglykol, Dipropylen-glykol, Polypropylenglykol, Dibutylenglykol und Polybutylenglykol. Weitere geeignete Polyesterpolyole sind durch Polykondensation herstellbar. So können difunktionelle und/oder trifunktionelle Alkohole mit einem Unterschuss an Dicarbonsäuren und/oder Tricarbonsäuren, oder deren reaktiven Derivaten, zu Polyesterpolyolen kondensiert werden. Geeignete Dicarbonsäuren sind beispielsweise Adipinsäure oder Bernsteinsäure und ihre höheren Homologen mit bis zu 16 C-Atomen, ferner ungesättigte Dicarbonsäuren wie Maleinsäure oder Fumarsäure sowie aromatische Dicarbonsäuren, insbesondere die isomeren Phthalsäuren, wie Phthalsäure, Isophthalsäure oder Terephthalsäure. Als Tricarbonsäuren sind beispielsweise Zitronensäure oder Trimellithsäure geeignet. Die genannten Säuren können einzeln oder als Gemische aus zwei oder mehreren davon eingesetzt werden. Besonders geeignet sind Polyesterpolyole aus mindestens einer der genannten Dicarbonsäuren und Glyzerin, welche einen Restgehalt an OH-Gruppen aufweisen. Besonders geeignete Alkohole sind Hexandiol, Ethylenglykol, Diethylenglykol oder Neopentylglykol oder Gemische aus zwei oder mehreren davon. Besonders geeignete Säuren sind Isophthalsäure oder Adipinsäure oder deren Gemische. Polyesterpolyole mit hoher mittlerer zahlengemittelten molaren Masse Mₙ insbesondere im Bereich von 5000 g / mol umfassen beispielsweise die Umsetzungsprodukte von polyfunktionellen, vorzugsweise difunktionellen Alkoholen (gegebenenfalls zusammen mit geringen Mengen an trifunktionellen Alkoholen) und polyfunktionellen, vorzugsweise difunktionellen Carbonsäuren. Anstatt freier Polycarbonsäuren können (wenn möglich) auch die entsprechenden Polycarbonsäureanhydride oder entsprechende Polycarbonsäureester mit Alkoholen mit vorzugsweise 1 bis 3 C-Atomen eingesetzt werden. Die Polycarbonsäuren können aliphatische, alicyclische, aromatische oder heterocyclische Polycarbonsäuren sein. Sie können gegebenenfalls substituiert sein, beispielsweise durch Alkylgruppen, Alkenylgruppen, Ethergruppen oder Halogene. Als Polycarbonsäuren sind beispielsweise Bernsteinsäure, Adipinsäure, Korksäure, Azelainsäure, Sebacinsäure, Phthalsäure, Isophthalsäure, Terephthalsäure, Trimellithsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Dimerfettsäure oder Trimerfettsäure oder Gemische aus zwei oder mehr davon geeignet. Gegebenenfalls können untergeordnete Mengen an monofunktionellen Fettsäuren im Reaktionsgemisch vorhanden sein. Die Polyester können gegebenenfalls einen geringen Anteil an Carboxyendgruppen aufweisen. Aus Lactonen, beispielsweise epsilon-Caprolacton oder Hydroxycarbonsäuren, beispielsweise omega-Hydroxycapronsäure, erhältliche Polyester, können ebenfalls eingesetzt werden.

Auch können die vorgenannten Diole oder Polyole gemischt werden. Dabei ist die Verträglichkeit zu berücksichtigen. Bevorzugt werden im Zusammenhang mit der vorliegenden Erfindung aliphatische Polyesterpolyole eingesetzt.

Gemäß der vorliegenden Erfindung wird ein erfindungsgemäßer Polyurethangelschaum erhalten, indem mindestens ein Polysaccharid C, das mindestens eine Uronsäure umfasst, zusammen mit einer Isocyanatkomponente A und einer Polyolkomponente B zur Reaktion gebracht wird. Im Zusammenhang mit der vorliegenden Erfindung soll dabei unter einem Polysaccharid eine makromolekulare Verbindung verstanden sein, die aus mindestens zehn glycosidisch miteinander verknüpften Monosacharid-Molekülen besteht. Damit im Zusammenhang soll unter einem Polysaccharid, das mindestens eine Uronsäure oder ein Salz hiervon umfasst, ein Polysaccharid verstanden sein, das aus mindestens neun Monosacharid-Molekülen und einem Uronsäure-Molekül besteht, wobei diese Bestandteile glycosidisch verknüpft sind. Des Weiteren ist eine Uronsäure ein Monosaccharid, dessen primäre Alkoholfunktion (-CH₂OH) zu einer Carboxyfunktion (-COOH) oxidiert ist. Eine Uronsäure (Aldehydsäure) weist damit die allgemeine Formel (II) auf.

O=CH-[CH(OH)]*ₙ*-COOH mit (*n* ≥ 2) (II)

Insbesondere werden erfindungsgemäß solche Uronsäuren eingesetzt, für die n = 5 oder 6 gilt. Damit sind gemäß einem weiterführenden Aspekt der vorliegenden Erfindung insbesondere solche Polysaccharide einzusetzen, die mindestens eine Pentuoronsäure der allgemeinen Formel (III) oder eine Hexuronsäure der allgemeinen Formel (IV) umfassen.

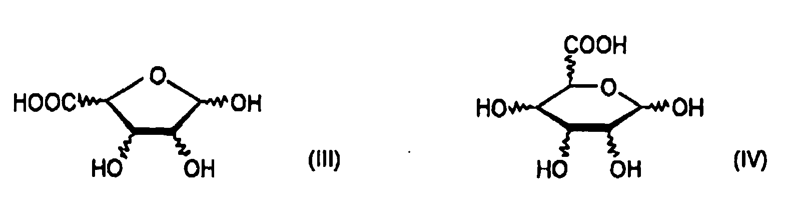

Gemäß einem weiterführenden Gedanken der vorliegenden Erfindung können insbesondere auch solche Polysaccharide C zur Reaktion gebracht werden, die als Uronsäure Guluronsäure oder ein Salz hiervon, Mannuronsäure oder ein Salz hiervon, Galacturonsäure oder ein Salz hiervon, Glucuronsäure oder ein Salz hiervon, Iduronsäure oder ein Salz hiervon, oder Mischungen dieser Säuren oder deren Salze umfassen.

Insbesondere kann jedoch auch vorgesehen sein, dass das Polysaccharid C ausgewählt wird aus der Gruppe Alginsäure oder deren Salze, Hyaluronsäure oder deren Salze, Glucosaminoglycane oder deren Salze, Xanthane oder deren Salze, oder Mischungen dieser Polysaccharide oder deren Salze. Diese Polysacharide weisen in unterschiedlichen Mengen Uronsäuren auf. So weisen beispielsweise Glycosaminoglycane als natürlich vorkommende Polysacharide1-4 verknüpfte Einheiten von Disachariden auf, die als Uronsäure Glucuronsäure oder Iduronsäure umfassen.

Ganz besonders bevorzugt können als Polysacharide C Alginsäure oder deren Salze eingesetzt werden. Alginsäure weist eine Struktur auf, die mit der allgemeinen Formel (V) wiedergegeben ist. Damit besteht Alginsäure im wesentlichen aus Manuronsäure und Guluronsäure.

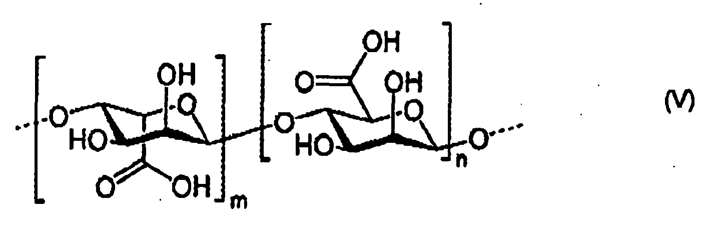

Hierbei bedeuten:
n, m unabhängig voneinander: n, m = 120 bis 1.000.

Insbesondere werden gemäß der vorliegenden Erfindung solche Alginsäuren gemäß Formel (V) bevorzugt, für die unabhängig voneinander n, m = 200 bis 1.000 gilt. Ganz besonders bevorzug werden solche Alginsäuren eingesetzt, für die unabhängig voneinander n, m = 200 bis 1.000, besonders bevorzugt n, m = 400 bis 1.000 und ganz besonders bevorzugt n, m = . 600 bis 1.000 gilt.

Weiterhin bevorzugt können als Polysacharide C Calziumalginate, Natriumalginata, Natrium-Calziumalginate oder Mischungen hiervon verwendet werden. Die Gerüstkette dieser Polysaccharide bestehen aus nahezu 100 % Guluronsäure und Mannuronsäure. Durch den hohen Anteil an Uronsäuren kann somit ein Polyurethangelschaum zur Verfügung gestellt werden, der besonders gut zur Verwendung als Wundkontaktschicht geeignet ist. Diese Polyurethangelschäume weisen eine Porenstruktur auf, die einen Durchtritt an Wundexsudat besonders begünstigt.

In der Regel weisen die im Rahmen der vorliegenden Erfindung einsetzbaren Polysaccharide C, insbesondere eingesetzte Alginsäuren oder deren Salze, eine mittlere zahlengemittelte molare Masse von etwa 2.000 g/ mol bis etwa 300.000 g/ mol, vorzugsweise etwa 5.000 g / mol bis etwa 300.000 g/ mol, besonderes bevorzugt etwa 10.000 g / mol bis etwa 300.000 g / mol, besonders bevorzugt etwa 10.000 g / mol bis etwa 250.000 g / mol und ganz besonders bevorzugt 100.000 g / mol bis etwa 250.000 g / mol auf.

Gegebenenfalls kann der erfindungsgemäße Polyurethangelschaum mittels weiterer Hilfs- und Zusatzstoffe hergestellt werden. Als Hilfs- und Zusatzstoffe können gemäß der vorliegenden Erfindung insbesondere Katalysatoren, Weichmacher, Stabilisatoren wie Antioxidantien oder Photostabilisatoren, Tackifier, Farbstoffe, Füllstoffe, Verdicker oder Rheologieadditive eingesetzt werden.

Als Katalysatoren zur Herstellung erfindungsgemäßen Polyurethangelschäume können hierbei insbesondere Wismut(III)-Carboxylate auf Basis linearer, verzweigter, gesättigter oder ungesättigter Carbonsäuren mit 2 bis 18, vorzugsweise 6 bis 18 C-Atomen eingesetzt werden. Weiterhin bevorzugt sind Bi(III)-Salze verzweigter gesättigter Carbonsäuren mit tertiären Carboxygruppen, wie der 2,2-Dimethyl-Octansäure (z.B. Versatic-Säuren, Shell Deutschland). Gut geeignet sind Zubereitungen dieser Bi(III)-Salze in überschüssigen Anteilen dieser Carbonsäuren. Hervorragend bewährt hat sich eine Lösung von 1 mol des Bi(III)-Salzes der Versatic 10-Säure (2,2-Dimethyloctansäure) in einem Überschuss von 3 mol dieser Säure mit einem Bi-Gehalt von ca. 17%. Diese Katalysatoren können insbesondere in der polymeren Polyolkomponente B vorgelegt werden. Hierbei werden die Katalysatoren bevorzugt in Mengen von 0,03 bis 0,5 Gew.-% bezogen auf die polymere Polyolkomponente B eingesetzt.

Als Weichmacher werden beispielsweise Phthalsäurederivate eingesetzt, beispielsweise Phthalsäureester, welche 6 bis 12 Kohlenstoffatome.aufweisen und mit einem linearen Alkanol verestert wurden, z.B. Dioctylphthalat. Polyethylengykole und deren Derivate, pflanzliche und tierische Öle, wie Glycerinester von Fettsäuren und deren Polymerisationsprodukte und Benzoatverbindungen (Benzoatweichmacher, beispielsweise Sucrosebenzoat, Diethylenglykoldibenzoat und/oder Diethylenglykolbenzoat, bei dem etwa 50 bis etwa 95% aller Hydroxylgruppen verestert worden sind, Phosphat-Weichmacher, beispielsweise t-Butylphenyldiphenylphosphat, Polyethylenglykole und deren Derivate, beispielsweise Diphenylether von Poly(ethylenglykol), flüssige Harzderivate, beispielsweise der Methylester von hydriertem Harz sind ebenfalls als Weichmacher geeignet. Besonders bevorzugt sind aliphatische Diester wie Adipin- oder Sebacindinonylester.

Zu den im Rahmen der Erfindung eingesetzten Stabilisatoren (Antioxidantien) zählen sterisch gehinderte Phenole wie BHT (2,6-Di-*tert*-butyl-4-methylphenol), Irganox® 1010, 1076, 1330, 1520 (Ciba Speciality Chemicals) sowie Toropherole. Besonders bevorzugt eingesetzt wird Vitamin E (alpha-Tocopherol). Ebenfalls können polyfunktionelle Phenole sowie schwefel- und phoshorhaltige Verbindungen und/oder 1,3,5-Trimethyl-2,4,6-tris(3,5-di-tert-butyl-4-hydroxybenzyl)benzol; Pentaerythrittetrakis-3-(3,5-di-tert-butyl-4-hydroxyphenyl)propionat; n-Octadecyl-3,5-di-tert-butyl-4-hydroxyphenyl)propionat; 4,4-Methylenbis(2,6-di-tert-butylphenol); 4,4-Thiobis(6-tert-butyl-o-cresol); 2,6-Di-tert-butylphenol-, 6-(4-Hydroxyphenoxy)-2,4-bis(n-octylthio)-1,3,5-triazin; Di-n-Octadecyl-3,5-di-tert-butyl-4-hydroxybenzyl-phos-phonate; 2-(n-Octylthio)ethyl-3,5-di-tert-butyl-4-hydroxy-ben-zoat; und Sorbithexa[3-(3,5-di-tertbutyl-4-hydroxy-phenyl)-propionat] zum Einsatz kommen. Als Photostabilisatoren sind zum Beispiel Tinuvin®-Produkte. (Ciba Speciality Chemicals), Benzotriazol-Verbindungen, Salicylate, substituierte Tolyl- und Metall-Chelat-Verbindungen geeignet, wobei Benzotriazol-Derivate bevorzugt werden. Kombinationen der oben genannten Verbindungen sind auch möglich. Die üblicherweise eingesetzten Mengen sind zwischen 0,1 und 10 Gew.%. Die Antioxidantien werden bevorzugt in Mengen von 0,15 bis 0,5 Gew.-% bezogen auf die polymere Polyolkomponente B eingesetzt.

Für die Einstellung bestimmter Eigenschaften des Polyurethangelschaums können fachüblich weitere Zusatzstoffe verwendet werden. Darunter fallen beispielsweise Farbstoffe wie Titandioxid, Füllstoffe wie Talkum, Kreide, Ton und dergleichen. Es ist ebenfalls möglich, bestimmte hydrophile Polymere einzubauen, beispielsweise, PVOH (Polyvinylalkohol), Polyvinylpyrrolidon, Hydroxypropylcellulose, Polyvinylmethylether und Celluloseester, speziell deren Acetate mit geringem Substitutionsgrad. Diese können die Benetzbarkeit des Polyurethangelschaums erhöhen. Unter Füllstoffen werden die in der Polyurethanchemie üblicherweise eingesetzten Füllstoffe verstanden. Darunter zählen auch Zinkoxid, Titanoxid und Kieselsäurederivate (z.B. Aerosile® (Degussa)). Als weitere Zusatzstoffe seien beispielsweise die Kurzfasern auf organischer oder anorganischer Basis (z.B. Textilfasern) genannt.

Um die Benetzung des Untergrundes zu erhöhen können dem Polyurethangelschaum übliche . Netzmittel zugegeben werden: beispielsweise Poloxamere (Copolymer von Polyoxyethylene and Polyoxypropylene), Sorbitanester, Fettsäuren wie Span® (Sigma-Aldrich), Ester von Polyoxyethylenesorbitan und Fettsäuren, wie Polysorbate oder Polysorbate® (Spectrum Chemical), Polyoxyethylierte hydrierte Castor Öle wie etwa Cremophor® (BASF), Polyoxyethylene Stearate, z.B. Myrj® (Uniqema) oder jede Kombination dieser Netzmittel. Vorzugsweise ist als Netzmittel ein Polysorbat einzusetzen.

Zusätzlich kann der Polyurethangelschaum Tackifierharze enthalten. Es können natürliche, modifizierte natürliche und synthetische Harze eingesetzt werden, typischerweise mit einer mittleren zahlengemittelten molaren Massen bis zu 1.500 g / mol. Die Kompatibilität der Harze mit den weiteren Komponenten ist jeweils in fachüblichen Routineversuchen zu prüfen. Beispielsweise sind Kohlenwasserstoffharze geeignet, insbesondere C5- bis C9-Harze, vorzugsweise mit C5-Harzen modifizierte C9-Harze, und dergleichen. Die gesamten Kohlenwasserstoffharze können teil- oder vollhydriert sein. Ebenfalls zum Einsatz kommen Naturharze wie Balsamharz oder Tallharz. Die genannten Harze können auch verestert werden mit entsprechenden polyfunktionellen Alkoholen wie Pentaerythritester, Glycerinester, Diethylenglykolester, Triethylenglykolester oder Methylester und so eingesetzt werden. Bekannte Handelsprodukte sind z.B. "Staybelite" ster 10, "Foral" 85-105, "Hercolyn" D, "Alresen" 214 R, "Alresen" 191 R, "Aresen" 500 R 80 und "Cellolyn" 21 s. Polyterpenharze wie auch die Terpenphenolharze können ebenfalls als Tackifierharze einformuliert werden wie auch die synthetischen Harze: Keton-, Kumaron- und Indenharze und auch KohlenwasserstoffHarze sind auch möglich z.B. unter Handelsnamen wie "Ketonharz" N, "Lutonal" J 30, "Lutonal" J 60, "Vinnapas" B 17, "Vinnapas" 50 V 1, Kohlenwasserstoffharz 95 KW 10, KW 20 and KW 30. Polyvinylether ist auch ein wirksamer Tackifier. Acrylatharze können ebenfalls alleine oder in Mischungen mit obengenanten Tackifiern eingesetzt werden.

Gemäß einer besonders bevorzugten Ausführungsform ist insbesondere auch ein Polyurethangelschaum und eine Wundauflage umfassend einen Polyurethangelschaum insbesondere als Wundkontaktschicht Gegenstand der vorliegenden Erfindung, wobei der Polyurethangelschaum aus den folgenden Komponenten erhalten wird:
a) eine Isocyanatkomponente A mit einer Funktionalität f von f_{A} ≤ 3,
b) eine polymere Polyolkomponente B mit einer Funktionalität f von f_{B} ≤ 6,
c) ein Polysaccharid C, das mindestens eine Uronsäure oder ein Salz hiervon umfasst, und
d) Hilfs- und Zusatzstoffe
wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht und
wobei die Hilfs- und Zusatzstoffe ausgewählt werden aus der Gruppe der Katalysatoren, Stabilisatoren und Antioxidantien. Insbesondere werden hierbei als Hilfs- und Zusatzstoffe ein Bi(III)-Carboxylat-Katalysator und Vitamin E eingesetzt.

Diese somit erhaltenen Polyurethangelschäume können darüber hinaus haftklebende Eigenschaften aufweisen. Gemäß einer weiteren Ausführung der vorliegenden Erfindung ist damit insbesondere auch ein haftklebender Polyurethangelschaum und eine Wundauflage, die einen haftklebenden Polyurethangelschaum, insbesondere als Wundkontaktschicht, umfasst, Gegenstand der vorliegenden Erfindung.

Dabei handelt es sich bei dem klebenden Polyurethangelschaum um einen Gelschaum, der eine schwache bis mäßig starke Haftklebrigkeit auf menschlicher Haut oder Gewebe aufweist. Insbesondere weist eine erfindungsgemäße Wundauflage eine Klebkraft von 0,05 bis 5 N/ 25 mm, insbesondere von 0,05 bis 3 N/ 25 mm und ganz besonders bevorzugt von 0,05 bis 2 N/ 25 mm auf. Hierbei wird die Klebkraft gemäß Test 1 (vgl. Testmethode) gegen Stahl unter einem Abzugswinkel von 90 ° gemessen.

Als Wundkontaktschicht ist gemäß der vorliegenden Erfindung eine Schicht zu verstehen, die eine erste und eine zweite Seite aufweist, wobei die erste Seite im anwendungsgerechten Zustand der Wundauflage einen direkten Kontakt zu einer Wunde bildet. Hinsichtlich der Form und Ausmaße der Wundkontaktschicht sind hierbei keine Grenzen gesetzt. Diese Wundkontaktschicht kann in Bezug auf eine absorbierende Schicht vollflächig oder diskontinuierlich sein und/ oder eine gleichmäßige oder eine profilierte Schichtdicke aufweisen und/ oder regelmäßige oder unregelmäßige Muster aufweisen.

Gemäß der vorliegenden Erfindung soll die Wundkontaktschicht mit der absorbierenden Schicht verbunden sein. Hiermit soll verstanden sein, dass die Wundkontaktschicht in mindestens einem Bereich ihrer zweiten Seite mit einer ersten, im anwendungsgerechten Zustand zu einer Wunde weisenden Seite der absorbierenden Schicht in direktem Kontakt steht. Besonders bevorzugt umfasst die Wundauflage eine Wundkontaktschicht, deren zweite Seite vollflächig mit der ersten Seite der absorbierenden Lage verbunden ist. Alternativ hierzu kann die Wundkontaktschicht auch integral mit der absorbierenden Schicht verbunden sein. Hiermit soll verstanden sein, dass die beiden miteinander verbundenen und aneinandergrenzenden Schichten an ihren Grenzflächen eine Übergangsschicht bilden, die nicht separiert werden kann. Durch diese integral verbundenen Schichten wird darüber hinaus ein Laminat zur Verfügung gestellt, das keine separierbaren Schichten umfasst, die chemisch und/ oder physikalisch miteinander verbunden sind.

Dem entsprechend ist eine mehrschichtige Wundauflage umfassend eine Trägerschicht, eine absorbierende Schicht mit einer ersten und einer zweiten Seite und eine Wundkontaktschicht mit einer ersten und einer zweiten Seite, wobei die zweite Seite der Wundkontaktschicht vollflächig mit der ersten Seite der absorbierenden Schicht verbunden ist und ein Polyurethangelschaum umfasst, ebenfalls Gegenstand der vorliegenden Erfindung. Besonders bevorzugt weist diese Wundauflage eine Wundkontaktschicht auf, die in Bezug auf die erste Seite der absorbierenden Schicht vollflächig ist. Ganz besonders bevorzugt weist diese Wundauflage eine Wundkontaktschicht auf, die integral mit der absorbierenden Schicht verbunden ist und die insbesondere vollflächig in Bezug auf die erste Seite der absorbierenden Schicht ist.

Schließlich kann auch vorgesehen sein, dass die Wundauflage eine Wundkontaktschicht aufweist, die in Bezug auf die erste Seite der absorbierenden Schicht nicht vollflächig ist, sondern einzelne Bereiche der absorbierenden Schicht ausspart, beispielsweise um hier einen Klebeauftrag zur Befestigung der Wundauflage auf der Haut eines Patienten anzubringen. Alternativ kann die Wundauflage auch nicht vollflächig in Bezug auf die absorbierende Schicht ausgestaltet sein, indem eine diskontinuierliche Wundkontaktschicht gewählt wird, die regelmäßige oder unregelmäßig angeordnete Aussparungen vorsieht. Diese Aussparungen stellen einen verbesserten Durchgang von Wundflüssigkeiten von der Wunde zur absorbierenden Schicht zur Verfügung.

Als besonders vorteilhafte Ausführungen haben sich weiterhin Wundauflagen gezeigt, die eine Wundkontaktschicht aufweisen, deren Schichtdicke weniger als 1000 µm aufweist. Insbesondere weist damit eine erfindungsgemäße Wundauflage eine Wundkontaktschicht mit einer Schichtdicke von 10 bis 1000 µm, insbesondere von 10 bis 500 µm und ganz besonders bevorzugt von 10 bis 250 µm auf. Wundauflagen mit solchen Schichtdicken zeigen einerseits keine Wundverklebung und andererseits die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Als absorbierende Schicht kann jedes heute in der modernen Wundbehandlung übliche, als absorbierende Schicht einzusetzende Material Verwendung finden. Hierbei sind insbesondere solche Materialien zu nennen, die in der feuchten Wundtherapie angewendet werden können. Ganz besonders sind jedoch solche absorbierenden Schichten bevorzugt, die sowohl Wundsekret aufnehmen und somit absorbierend wirken, als auch Feuchtigkeit an die Wunde abgeben. Weiterhin bevorzugt sind solche absorbierenden Schichten, die transparent oder transluzent sind. Gemäß einer besonders bevorzugten Ausgestaltung der vorliegenden Erfindung umfasst die Wundauflage als absorbierende Schicht einen hydrophilen PolymerSchaum, ein absorbierendes Vlies oder Nonwoven, eine mindestens ein Hydrocolloid umfassende Polymermatrix, einen gefriergetrockneten Schaum oder Kombinationen hiervon.

Wird als absorbierende Schicht ein hydrophiler Polymerschaum verwendet, so hat sich gezeigt, dass sich die Wundauflage besonders gut an eine zu behandelnde Wunde anpasst. Als Polymerschäume sind hierbei insbesondere hydrophile Polyurethan-Schäume geeignet. Dem gemäß umfasst eine besonders bevorzugte Wundauflage eine absorbierende Schicht aus einem hydrophilen Polyurethan-Schaum. Diese Polyurethan-Schäume weisen eine freie Absorption von mindestens 10 g/ g, insbesondere mindestens 12 g/g und ganz besonders bevorzugt von mindestens 15 g/ g auf, wobei die freie Absorption gemäß der DIN EN 13726-1 (2002) bestimmt wird. Weiterhin bevorzugt weisen diese Schäume eine Porengröße von durchschnittlich weniger als 1000 µm, insbesondere 200 bis 1000 µm und ganz besonders bevorzugt 200 bis 700 µm auf. Hierbei kann vorgesehen sein, dass die Porengröße an einer ersten Oberfläche der absorbierenden Schicht im Wert verschieden ist von der Porengröße einer zweiten Oberfläche der absorbierenden Schicht. Weiterhin bevorzugte hydrophile Polyurethan-Schäume weisen eine Dichte von weniger als 150 kg/m³, insbesondere weniger als 140 kg/ m³ und ganz besonders bevorzugt 70 bis 120 kg/ m³ auf.

In einer erfindungsgemäßen Alternative umfasst die absorbierende Schicht insbesondere wasserunlösliche Fasern aus Cellulose, insbesondere weitgehend delignifizierte technische Zellstofffasern, insbesondere Holzzellstofffasem, insbesondere mit einer Faserlänge von kleiner 5 mm. Das Fasermaterial kann auch hydrophiles Fasermaterial aus regenerierter Cellulose, Carboxymethylcellulose, Carboxyethylcellulose, Hydoxymethylcellulose oder Hydroxyethylcellulose enthalten. Es kann auch vorgesehen sein, eine Fasermischung aus Cellulose-, regenerierter Cellulose-, Carboxymethylcellulose-, Carboxyethylcellulose-, Hydoxymethylcellulose- oder Hydroxyethylcellulose-Fasem und Fasern aus Polyethylen, Polypropylen oder Polyester vorzusehen. In einer ganz besonders bevorzugten Ausführungsform umfasst die absorbierende Schicht eine Mischung aus Cellulosefasern, Polypropylenfasern und partikelförmigem superabsorbierendem Polymer vorzugsweise aus vernetztem Natrium-Polyacrylat.

Als Trägerschicht können verschiedene Materialien eingesetzt werden. Üblicherweise werden in Wundauflagen textile Trägermaterialien, Nonwoven, Polymerfilme oder Polymerschäume eingesetzt. Als Trägerschicht einer erfindungsgemäßen Wundauflage können insbesondere Polymerfilme oder Polymerschäume eingesetzt werden. Ganz besonders bevorzugt sind polymerfilme, die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren, Polyacrylat oder Polymethacrylat gefertigt werden. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen.

Die Wasserdampfdurchlässigkeit des Polymerfilms der Wundauflage weist vorzugsweise mindestens 750 g/ m²/24 Std., insbesondere mindestens 1000 g/ m²/24 Std. und ganz besonders bevorzugt mindestens 2000 g/ m²/24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnde Fläche umgebenden Haut austreten kann. Als besonders bevorzugt sind solche Klebstoffe zu betrachten, die in einem dünnen Auftrag von 20 bis 35 g/ m² zusammen mit dem Film eine Wasserdampfdurchlässigkeit von mindestens 800 g/ m²/24 Std. und vorzugsweise von mindestens 1000 g/ m²/24 Std. (gemessen nach DIN EN 13726) aufweisen.

In einer alternativen und besonders bevorzugten Ausführung einer erfindungsgemäßen Wundauflage kann die Trägerschicht auch aus einem Polymerschaum gefertigt sein. Insbesondere ist in dieser Ausgestaltung vorgesehen, dass der Polymerschaum ein Polyurethan-Schaum ist. Diese Polyurethanschäume sind aus im Wesentlichen geschlossenporigen Polyurethanschäumen gefertigt und weisen insbesondere eine Dicke von 0,01 bis 2 mm auf. Dabei zeigen sich im Wesentlichen geschlossenporige Polyurethanschäume als besonders vorteilhaft, da diese Schäume sowohl wasserdampfdurchlässig sind als auch eine Barriere für Schmutz und Keime darstellen. Dieses Trägermaterial weist besonders bevorzugt eine Wasserdampfdurchlässigkeit von mindestens 750 g/ m²/24 Std., insbesondere mindestens 1000 g/ m²/24 Std. und ganz besonders bevorzugt mindestens 1200 g/ m²/24 Std. auf (gemessen nach DIN EN 13726) auf.

In einer weiterhin bevorzugten Ausführung weist die erfindungsgemäße Wundauflage als Trägerschicht und als absorbierende Schicht ein Laminat aus zwei verschiedenen Polyurethan-Schäumen auf. Insbesondere besteht diese Wundauflage aus einem Laminat aus zwei verschiedenen Polyurethan-Schäumen, das auf der im anwendungsgerechten Zustand der Wundauflage zur Wunde weisenden Seite mit einer Wundkontaktschicht aus einem haftklebenden, Polyurethangelschaum beschichtet ist. Damit ist insbesondere eine Wundauflage bestehend aus einer Trägerschicht aus einem ersten hydrophoben, für Wasserdampf durchlässigen Polyurethan-Schaum, einer absorbierenden Schicht aus einem hydrophilen, absorbierenden Polyurethan-Schaum und einer mit der absorbierende Schicht verbundenen, insbesondere integral verbundenen Wundkontaktschicht, die ein von dem hydrophilen Schaum verschiedenen, haftklebenden Polyurethangelschaum umfasst, Gegenstand der vorliegenden Erfindung. Insbesondere umfasst diese Wundauflage auch eine absorbierende Schicht, die eine höhere freie Absorption aufweist als die Wundkontaktschicht. Diese Wundauflage ist besonders vorteilhaft als unterstützende Maßnahme in der modernen Wundbehandlung einzusetzen, da durch die Wundkontaktschicht keine Wundverklebung stattfindet, keine Mazeration der die Wunde umgebenden Haut stattfindet, eine schnelle Aufnahme von der Wunde abgegebenen Wundflüssigkeiten stattfindet und eine Fixierung der Wundauflage auf der die Wunde umgebenden Haut vorgenommen werden kann.

In einer alternativen Form einer erfindungsgemäßen Wundauflage umfasst eine Wundkontaktschicht weiterhin mindestens ein die Wundheilung aktiv unterstützendes Mittel, insbesondere ein antimikrobielles Mittel, ein Vitamin oder Provitamin, eine Fettsäure oder Fettsäureester oder ein den Gewebeaufbau aktiv förderndes Mittel.

Gemäß einer besonders bevorzugten Alternative umfasst die Wundkontaktschicht weiterhin mindestens ein antimikrobielles Mittel. Insbesondere sind hierbei antimikrobielle Metalle oder deren Salze, insbesondere Silber oder dessen Salze geeignet. In einer besonders bevorzugten Ausführung umfasst die Wundkontaktschicht ein antimikrobielles Mittel und ein Trägermaterial für das antimikrobielle Mittel. Hierbei können bevorzugt als Trägermaterial ein Nonwoven oder ein textiles Material wie Gestrick, Gewirk oder Gewebe verwendet werden, das mit einem antimikrobiell wirkenden Metall vorzugsweise Silber oder Silbersalze beschichtet ist. Dabei ist es besonders vorteilhaft, wenn dar Polyurethangelschaum wasserfrei ist.

Gemäß einem weiterführenden Gedanken der vorliegenden Erfindung, ist auch eine mehrschichtige Wundauflage umfassend eine Trägerschicht, eine absorbierende Schicht, eine Wundkontaktschicht und eine Verteilerschicht, wobei die Wundkontaktschicht einen Polyurethangelschaum umfasst, ebenfalls Gegenstand der vorliegenden Erfindung. Insbesondere ist die absorbierende Schicht mit der Wundkontaktschicht verbunden. Eine solche Wundauflage weist besonders vorteilhaft eine Verteilerschicht zwischen der Trägerschicht und der absorbierenden Schicht auf, die insbesondere aus einem hydrophilen Polyurethan-Schaum besteht. Durch die Verteilerschicht wird erreicht, dass eine Verteilung der aufgenommen Wundflüssigkeiten über die gesamte Fläche der Wundauflage insbesondere oberhalb der absorbierenden Schicht ermöglicht wird, das heißt das die Aufnahme der Wundflüssigkeiten nicht nur in z-Richtung (von der Wunde weg in Richtung Trägerschicht), sondern auch in x-y-Richtung (über die Fläche der Wundauflage) erfolgt.

Gemäß einem weiteren Aspekt ist auch ein Verfahren zur Herstellung eines Polyurethangelschaums, insbesondere ein Verfahren zur Herstellung des oben beschriebenen Polyurethangelschaums, insbesondere zur Verwendung als Wundkontaktschicht in einer Wundauflage, Gegenstand der vorliegenden Erfindung. Damit ist auch ein Verfahren zur Herstellung eines Polyurethangelschaums zur Verwendung als Wundkontaktschicht Gegenstand der vorliegende Erfindung, das dadurch gekennzeichnet ist, dass der Polyurethangelschaum aus mindestens folgenden zur Reaktion gebrachten Komponenten erhalten wird:
a) eine Isocyanatkomponente A mit einer Funktionalität f von f_{A} ≤ 3,
b) eine polymere Polyolkomponente B mit einer Funktionalität f von f_{B} ≤ 6 und
c) ein Polysaccharid C, das mindestens eine Uronsäure oder ein Salz hiervon umfasst,
wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht.

Gemäß einer weiteren Ausführung der vorliegenden Erfindung kann dabei auch vorgesehen sein, dass das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 3 bis 1:30, insbesondere 1 : 4 bis 1 : 30, insbesondere 1 : 4 bis 1 : 20 und ganz besonders bevorzugt 1 : 5 bis 1: 20 entspricht.

Hervorzuheben ist hierbei, dass sowohl die polymere Polyolkomponente B als auch das Polysacharid C als Reaktionspartner mit der Isocyanatkomponente A reagieren. Hierbei kann das Polysacharid C mit der Isocyanatkomponente A einerseits mittels vorhandener Hydroxylgruppen unter Ausbildung einer Urethanbindung reagieren und andererseits mittels vorhandener Carboxygruppe der Uronsäure unter Ausbildung einer Amidbindung reagieren.

Diese Amidbindung erfolgt unter gleichzeitiger Freisetzung von gasförmigem Kohlenstoffdioxid, wodurch der Polyurethangelschaum ohne Einbringen von zusätzlichen Gasen erhalten wird.

Insbesondere ist damit auch ein Verfahren zur Herstellung eines haftklebenden Polyurethangelschaums Gegenstand der vorliegenden Erfindung.

Unabhängig hiervon wird dabei in einem ersten Verfahrensschritt die Polyolkomponente B und das Polysaccharid C zu einer Polyol-Polysaccharid-Dispersion vermischt. In einer weiter bevorzugten Ausführung des Verfahrens wird in einem ersten Verfahrensschritt die Polyolkomponente B und das Polysaccharid C zu einer Polyol-Polysaccharid-Dispersion vermischt und die somit hergestellte Polyal-Polysaccharid-Dispersion in einem zweiten Verfahrensschritt mit der Isocyanatkomponente A zur Reaktion gebracht. Hierbei können weitere Hilfs- und Zusatzstoffe in die Polyol-Polysaccharid-Dispersion eingemischt sein.

Besonders bevorzugt ist ein Verfahren zur Herstellung des Polyurethangelschaums, in dem das Verhältnis der Anzahl der Isocyanatgruppen der lsocyanatkomponente A zur Gesamtzahl der Carboxygruppen und Carboxylatgruppen in dem Polysaccharid C dem Verhältnis 1 : 0,5 bis 1 : 10 entspricht.

Darüber hinaus ist ein Verfahren zur Herstellung eines Polyurethangelschaums bevorzugt, in dem die Isocyanatkomponente A ausgewählt wird aus der Gruppe der aliphatischen Di- oder Polyisocyanate, alicyclischen Di- oder Polyisocyanate oder Mischungen hiervon. Besonders bevorzugt ist hierbei als Isocyanatkomponente A Isophorondiisocyanat, ein Isophorondiisocyanat-Prepolymer oder eine Mischungen hiervon einzusetzen, wobei das Isophorondiisocyanat-Prepolymer insbesondere erhältlich ist aus Isophorondüsacyanat und einem Polyetherpolyol D mit einer Funktionalität f von f_{D} ≤ 3. Ganz besonders bevorzugt ist hierbei ein Polyetherpolyol D ausgewählt wird aus der Gruppe der Polyethylenglykole, Polypropylenglykole, Polyethylen-Polypropylenglykole oder Mischungen hiervon, wobei das Polyetherpolyol D eine mittlere zahlengemittelte molare Masse Mₙ von mindestens Mₙ (D) = 2000 g/ mol und höchstens Mₙ (D) =10.000 g/ mol aufweist.

Unabhängig hiervon kann in dem Verfahren besonders bevorzugt als Polyolkomponente B verwendet werden, die eine Funktionalität f von 3 ≤ f_{B} ≤ 6 aufweist. Insbesondere kann als Polyolkomponente B ein Polyetherpolyol eingesetzt werden, das weiterhin bevorzugt eine Funktionalität f von 3 ≤ f_{B} ≤ 6 aufweist. Weiterhin bevorzugt kann als Polyolkomponente B ein Polyetherpolyol eingesetzt werden, das eine mittlere zahlengemittelte molare Masse Mₙ von 3.000 bis 10.000 g/ mol aufweist.

Gemäß einer Weiterbildung der Erfindung kann in dem Verfahren zur Herstellung eines Polyurethangelschaum ein Polysaccharid C verwendet werden, das als Uronsäure Guluronsäure oder ein Salz hiervon, Mannuronsäure oder ein Salz hiervon, Galacturonsäure oder ein Salz hiervon, Glucuronsäure oder ein Salz hiervon, lduronsäure oder ein Salz hiervon, oder Mischungen dieser Uronsäuren oder deren Salze umfasst. Insbesondere können als Polysaccharid C in dem Verfahren auch Polysaccharide verwendet werden, die ausgewählt werden aus der Gruppe Alginsäure oder deren Salze, Hyaluronsäure oder deren Salze, Glucosaminoglycane oder deren Salze, der Xanthane oder deren Salze, oder Mischungen dieser Polysaccharide oder deren Salze. Ganz besonders bevorzugt ist ein Verfahren, indem das Polysaccharid C ein Calziumalginat, ein Natriumalginat, ein Natrium-Calziumalginat oder eine Mischungen hiervon ist.

In Fortbildung der Erfindung ist auch ein Verfahren zur Herstellung einer Wundauflage Gegenstand der vorliegenden Erfindung. In dem Verfahren wird in einem ersten Verfahrensschritt ein Polyurethangelschaum hergestellt, indem mindestens folgende Komponenten zur Reaktion gebracht werden:
a) eine Isocyanatkomponente A mit einer Funktionalität f von f_{A} ≤ 3,
b) eine polymere Polyolkomponente B mit einer Funktionalität f von f_{B} ≤ 6 und
c) ein Polysaccharid C, das mindestens eine Uronsäure oder ein Salz hiervon umfasst,
wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht.

Insbesondere umfasst die Wundauflage den somit hergestellten Polyurethangelschaum als Wundkontaktschicht.

In einem zweiten Verfahrensschritt wird der fertige Polyurethanschaum auf eine Trägerschicht oder eine absorbierende Schicht aufgebracht, laminiert-oder übertragen. Hierbei wird der fertige Polyurethangelschaum insbesondere auf eine absorbierende Schicht aufgebracht, laminiert oder übertragen, wobei als absorbierende Schicht ein absorbierender hydrophiler Polymerschaum oder ein absorbierendes Textilmaterial ist.

Es kann jedoch auch vorgesehen sein, dass in einem ersten Verfahrenschritt zur Herstellung der Wundauflage ein Polyurethangelschaum direkt auf einer Trägerschicht oder einer absorbierenden Schicht gebildet wird, wobei die Trägerschicht oder die absorbierende Schicht Bestandteil der Wundauflage ist. Damit kann eine Wundauflage bereitgestellt werden, die eine absorbierende Schicht aufweist, die einen direkten Kontakt mit dem Polyuretnagelschaum aufweist.

Im Folgenden soll die Erfindung an Hand von Zeichnungen erläutert werden, ohne dass diese Zeichnungen eine bezüglich der Erfindung einschränkende Wirkung haben sollen. Dabei zeigen:
- Figuren 1 bis 3: jeweils alternative Ausführungen einer erfindungsgemäßen Wundauflage im Querschnitt.
- Figuren 4a und 4b: eine Messanordnung zur Bestimmung der Anfassklebrigkeit.
- Figuren 5 und 6: jeweils eine lichtmikroskopische Aufnahme eines erfindungsgemäßen Polyurethangelschaums.

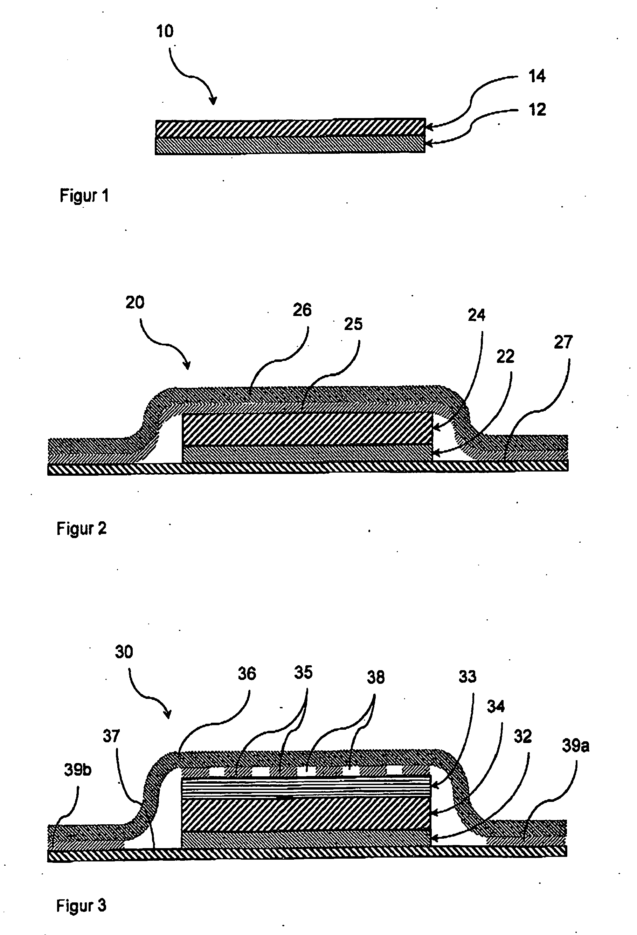

Mit Figur 1 ist der einfachste Aufbau einer erfindungsgemäßen Wundauflage (10) wiedergegeben. Die Wundauflage besteht aus einer Wundkontaktschicht (12) aus einem erfindungsgemäßen Polyurethangelschaum, der auf einem absorbierenden Vliesstoff aufgebracht ist. Durch die Beschichtung mit dem Polyurethangelschaum ist einerseits ein Verkleben mit der Wunde verhindert und andererseits eine Wundauflage bereitgestellt, die trotz Beschichtung eine gute und schnelle Aufnahme an Flüssigkeiten zeigt.

Mit Figur 2 ist eine erfindungsgemäße Wundauflage (20) als so genannter Inselverband gezeigt. Die Wundauflage besteht aus einem absorbierenden, hydrophilen Polyurethanschaum als absorbierende Lage (24), die auf der im anwendungsgerechten Zustand einer Wunde zugewandten Seite mit einer Wundkontaktschicht (22) aus einem erfindungsgemäßen haftklebenden Polyurethangelschaum vollflächig beschichtet ist. Dabei weist der Polyurethangelschaum einen direkten Kontakt mit dem absorbierenden Polyurethanschaum auf. Auf der im anwendungsgerechten Zustand der Wunde abgewandten Seite der absorbierenden Lage ist mittels eines vollflächig aufgebrachten Acrylat-Haftklebers (25) eine Trägerschicht (26) aus einem geschlossenzelligen Polyurethan-Schaum aufgebracht. Sowohl die Wundkontaktschicht (22) als auch der die Wundkontaktschicht allseits umgebende klebende Rand sind vor Gebrauch der Wundauflage mit einem silikonisierten Release-Papier (27) abgedeckt. Diese Wundauflage weist zwei verschieden stark haftende Haftklebezonen auf.

Mit Figur 3 ist ebenfalls eine erfindungsgemäße Wundauflage (30) als Inselverband gezeigt. Die Wundauflage besteht aus einer Wundkontaktschicht (32) aus einem erfindungsgemäßen Polyurethangelschaum, der vollflächig auf einen hydrophilen, absorbierenden Polyurethan-Schaum (34) aufgebracht und mit dem Schaum integral verbunden ist. Die absorbierende Schicht weist eine Schichtdicke von 5 mm auf, wobei der Polyurethan-Schaum eine Porengröße von 300 bis 900 µm aufweist. Auf der der Wundkontaktschicht gegenüberliegenden Oberfläche der absorbierenden Schicht ist zwischen der Trägerschicht (36) und der absorbierenden Schicht (34) eine weitere absorbierende Lage (33) aufgebracht. Diese absorbierende Lage (33) dient als Verteilerschicht für bereits durch die Wundkontaktschicht (32) und die absorbierende Schicht (34) aufgenommene Flüssigkeitsmenge. Die absorbierende Verteilerechicht ermöglicht eine gleichmäßige Verteilung der aufgenommenen Flüssigkeiten in x-y-Richtung, wogegen die absorbierende Schicht (34) als auch die Wundkontaktschicht (32) eine Absorption der Wundflüssigkeiten in z-Richtung, d.h. senkrecht zur Wundoberfläche gewährleisten. Die Trägerschicht (36) besteht aus einem dünnen für Wasserdampf sehr gut durchlässigen Polyurethan-Film, der eine Schichtdicke von 70 µm aufweist. Die Verteilerschicht besteht aus einem Verteilervlies aus Cellulosefasern, das mittels streifenförmig aufgebrachten Acrylat-Haftklebstoff (35) an der Trägerschicht (36) befestigt ist. Somit kann durch die von Acrylat-Klebstoff frei bleibenden Stellen (38) ein gegenüber einem vollflächig beschichteten Film verbesserter Wasserdampfaustausch mit der Umgebung stattfinden. Sowohl die Wundkontaktschicht (32) als auch der die Wundkontaktschicht allseits umgebende klebende Rand (39a, 39b) aus einem Acrylat-Haftklebstoff sind vor Gebrauch der Wundauflage mit einem silikonisierten Release-Papier (37) abgedeckt.

Hervorzuheben ist an dieser Stelle, dass die hier aufgeführten Merkmale der alternativen oder bevorzugten Ausgestaltungen der Erfindungen nicht auf die einzelnen Alternativen zu beschränken sind. Im Zusammenhang mit der vorliegenden Erfindung ist es vielmehr der Fall, dass eine Kombination von Ausgestaltungen bzw. eine Kombination jedes Einzelmerkmals einer alternativen Form mit Merkmalen einer anderen alternativen Ausgestaltung ebenso als erfindungsgemäßer Gegenstand zu sehen ist.

### Ausführungsbeispiele

### A) Testmethoden:

### 1) Klebkraft auf Stahl (90 ° Abzugswinkel) in Anlehnung an AFERA 5001) - Test 1

Das zu prüfende Muster wird vor der Prüfung 24 h im Normklima (23 °C, 50-% relative Feuchte) gelagert und danach 3 Proben von je 25 mm Breite und 100 mm Länge entnommen. Der Prüfkörper wird mit der Hand vorsichtig und dehnungsfrei auf Stahlplatten (nach DIN EN 1939) auflaminiert, wobei Luftblasenbildung vermieden wird. Auf die nicht klebende Oberseite des Musters wird ein handelsübliches Verstärkungsklebeband, das nicht dehnbar ist (z.B. Tesa 4104) aufgebracht, um die Dehnung des Schaums zu eliminieren. Der Prüfkörper wird mit Hilfe eines Tape Applicators D 427/1 der Firma Sondes Place Research Institute, Surrey England mit 20 N/ cm definiert angerollt. Die so präparierte Stahlplatte wird in die 90° Abzugsvorrichtung der Zug-Dehnungsmaschine Z-005 der Firma Zwick-Roell, Ulm Deutschland, eingelegt und das freie, über das Muster hinaus stehende Ende des Verstärkungsklebebandes in die obere Klemme eingespannt. Bei einer konstanten Abzugsgeschwindigkeit von 300 mm/ min wird der Kraftverlauf gemessen, der benötigt wird, um die Probe von der Stahlplatte zu lösen. Die Ermittlung der Klebekraft erfolgt durch ein geeignetes PC-Programm nach DIN 53 539 (Verfahren C).

### 2) Anfangsklebrigkeit - Test 2

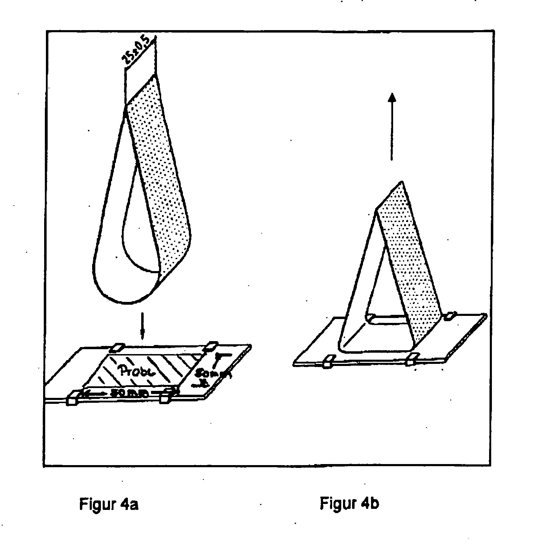

Als Tack wird die Anfangsklebrigkeit bezeichnet, die sich nach unmittelbaren Kontakt der Oberflächen ausbildet, im Gegensatz zur Klebkraft, die eine Kontaktzeit benötigt, um sich voll auszubilden. Mit Abbildung 4 ist die schematische Darstellung der Messanordnung zur Bestimmung der Anfangsklebrigkeit wiedergegeben. Für die Messung der Anfangsklebrigkeit wird die maximale Kraft gemessen, die erforderlich ist, um eine Schlaufe aus Polyesterfolie bei festgelegter Geschwindigkeit (300 mm/min) von einer klebenden Fläche, mit der sie vorher in Kontakt gebracht wurde, zu trennen. Die Probe wurde vor der Bestimmung 24 Stunden bei Normklima (23°C und 50% relative Luftfeuchtigkeit.) gelagert. Ein 50 x 50 mm großes Probenstück wurde mittels doppelseitigen Klebebands [410B von 3M] auf dem Probenteller befestigt. Die Polyesterschlaufe bestehend aus einer 50 µm dicken Polyesterfolie (Closure-Tape, Gerlinger Industries, Nördlingen, Deutschland), 175 mm Länge und 25 mm Breite, wurde vor Messbeginn auf die klebende Probe herabgelassen (vgl. Figur 4a). Die Polyesterschlaufe wird so weit herabgelassen, dass die Auflagefläche der Schlaufe auf dem Probenstück mindestens 25 x 25 mm beträgt (vgl. Figur 4b). Die Messung erfolgt direkt im Anschluss an das Herabsenken. Bei der Messung wird die maximale Kraft gemessen, die erforderlich ist, um die Schlaufe vollständig von der klebenden Wundauflage zu trennen. Für die Analyse wurde die Zugprüfmaschine Zwick Z005 [Zwick GmbH & Co.KG, Ulm - Deutschland) verwendet.

### 3) Durchtrittsgeschwindigkeit - Test 3

Zur Durchführung dieses Test wird eine Blutersatzlösung, bestehend aus einer Mischung von demineralisertem Wasser (56,4 Gew-%), Glyzerin (42,5 Gew-%, Sigma-Aldrich, Seelze - Deutschland), Natriumchlorid reinst (0,9 Gew-%, Sigma-Aldrich) und 0,2 Gew-% an dem Farbstoff Allura red (Sigma-Aldrich) verwendet. Es wird die Zeit bestimmt, die die Blutersatzlösung benötigt, um durch eine Polyurethangelschicht mit einer Schichtdicke von 180 µm auf einem Polyurethanschaum als absorbierendes Medium benötigt. Der Test wird mittels einer handelsüblichen Videokamera überwacht, wobei alle eingesetzten Mittel auf Raumklima einzustellen sind (23 °C, 50 % rel. Luftfeuchtigkeit). Dazu wurde ein Probenstück mit einem Durchmesser von ca. 5 cm ausgestanzt. Auf die plan nach oben liegende Polyurethangelschaumseite werden 0,5 ml der Blutersatzlösung mit Hilfe einer Pipette aufgebracht. Es wird danach die Zeit gemessen, die die Blutersatzlösung benötigt, um komplett durch die Polyurethangelschaumschicht in den Polyurethanschaum einzudringen.

### B) Erfindungsgemäße Polyurethangelschäume

### 1) Verwendete Komponenten zur Herstellung der Polyurethangelschäume

### i) Isocyanatkomponente A:

Für die Herstellung erfindungsgemäßer Polyurethangelschäume wurde als Isocyanatkomponente A ein Isophorondiisocyanat-Prepolymer gemäß Formel (I) eingesetzt, wobei n = 0 und m = 60 ± 5 bedeutet. Das Prepolymer weist als polymere Spacergruppe ein Polypropylenglycol auf. Das Prepolymer wurde aus Isophorondiisocyanat und einem Polypropylenglycol (Polyetherpolyol D) mit einem mittleren zahlengemittelten molaren Masse Mₙ (D) = 3500 g/ mol hergestellt. Das Prepolymer weist einen niedrigen Monomeranteil an Isophorondiisocyanat von weniger als 0,5 Gew.-% auf.

Das Prepolymer weist folgende Kennzahlen auf:
a) mittlere zahlengemittelte molare Masse Mₙ: Mₙ (A) = 4000 g/ mol
b) Funktionalität f: f_{A}= 2
c) mittlere Äquivalentmasse M(eq): M(eq)_{A} = 2000 g/ mol
d) Anzahl der Isocyanatgruppen pro kg (Z): Z_{A} = 500 mmol

### ii) polymere Polyolkomponente B

Als polymere Polyolkomponente B wurde ein Polyethylen-Polypropylenglycol (Polyetherpolyol) eingesetzt, das folgende Kennzahlen aufweist
a) mittlere zahlengemittelte molare Masse Mₙ: Mₙ (B) = 6400 g/ mol
b) Funktionalität f: f_{B} = 4
c) mittlere Äquivalentmasse M(eq): M(eq)_{B} = 1600 g/ mol
d) Anzahl der Isocyanatgruppen pro kg (Z): Z_{B} = 625 mmol

### iii) Polysaccharid C

Als Polysaccharid C wurde ein Calziumalginat (Fluka BioChemika Nr. 21054 - Sigma-Aldrich Chemie GmbH Buchs, Schweiz) eingesetzt. Das Polysacharid ist aus den Zuckersäuren (Uronsäuren) Manuronsäure und Guluronsäure aufgebaut und weist ca. 510 Einheiten des Calziumsalzes des Disaccharids (Guluronsäure-Manuronsäure - C₁₂H₁₈O₁₂Ca) pro Mol auf. Das Alginat weist folgende Kennzahlen auf:
a) mittlere zahlengemittelte molare Masse M: Mₙ (C) = 200.000 g/ mol
b) mittlere Äquivalentmasse M(eq)
   (bezogen auf Hydroxylgruppen und Carboxy-/ Carboxylatgruppen): M(eq)_{C1} = 19,43 g/ mol
b) mittlere Äquivalentmasse M(eq)
   (bezogen auf Carboxy-/ Carboxylatgruppen): M(eq)_{C2} =196,08 g/ mol

### iv) weitere Hilfs- und Zusatzstoffe

Als weitere Hilfs- und Zusatzstoffe wurde ein Bi(III)-Neodecanoat (Coscat 83^{®} - Vertellus Performance Materials Inc., Greensboro, NC, USA) und Vitamin E (Tocopherol - Merck KGaA, Darmstadt, Deutschland) eingesetzt.

Die Komponenten A und B können von der Firma Nolax AG (Sempach Station - Schweiz) unter dem Namen M 41.3032 bezogen werden, wobei die polymere Polyolkomponente B die weiteren Hilfs- und Zusatzstoffe Stabilisator und Katalysator umfasst.

### 2) Zusammensetzung und Herstellung der Polyurethanschäume und daraus hergestellte Wundauflagen

Die Polyurethangelschäume weisen die mit Tabelle 1 wiedergegebenen Zusammensetzungen auf.

**Tabelle 1: Zusammensetzung der Polyurethangelschäume**

| | Isocyanat A | | Polyol B | | Polysaccharid C | | Stabilisator | | Katalysator | |
|---|---|---|---|---|---|---|---|---|---|---|
| | [g] | Gew-% | [g] | GeW-% | [g] | Gew-% | [g] | Gew-% | [g] | Gew-% |
| PUALGS1 | 10,58 | 43,25 | 12,533 | 51,24 | 1,27 | 5,19 | 0,025 | 0,10 | 0,052 | 0,21 |
| PUALGS2 | 8,82 | 42,57 | 10,744 | 51,85 | 1,09 | 5,28 | 0,022 | 0,11 | 0,044 | 0,21 |
| PUALGS3 | 10,09 | 43,25 | 10,955 | 46,96 | 2,22 | 9,52 | 0,020 | 0,09 | 0,045 | 0,19 |
| PUALGS4 | 8,97 | 43,10 | 9,800 | 47,09 | 1,98 | 9,51 | 0,020 | 0,10 | 0,040 | 0,19 |
| PUALGS5 | 8,98 | 43,01 | 9,100 | 43,58 | 2,75 | 13,17 | 0,012 | 0,06 | 0,038 | 0,18 |
| PUALGS6 | 10,27 | 42,46 | 10,645 | 44,01 | 3,21 | 13,27 | 0,021 | 0,09 | 0,044 | 0,18 |
| PUALGS7 | 11,16 | 42,99 | 10,635 | 40,97 | 4,10 | 15,79 | 0,021 | 0,08 | 0,044 | 0,17 |
| PUALGS8 | 11,54 | 42,84 | 7,633 | 28,33 | 7,72 | 28,66 | 0,015 | 0,08 | 0,032 | 0,12 |

**Tabelle 2: Eingesetzte Äquivalente**

| | Anzahl NCO-Gruppen im Isocyanat A [mmol] | Anzahl OH-Gruppen im Polyol B [mmol] | Anzahl OH-Gruppen und COOH/COO^{Θ}-Gruppen im Alginat [mmol] | Anzahl COOH/COO^{Θ}-Gruppen im Alginat [mmol] |
|---|---|---|---|---|
| PUALGS1 | 5,29 | 7,83 | 19,43 | 6,48 |
| PUALGS2 | 4,41 | 6,72 | 16,68 | 5,56 |
| PUALGS3 | 5,05 | 6,85 | 33,97 | 11,32 |
| PUALGS4 | 4,49 | 6,13 | 30,29 | 10,10 |
| PUALGS5 | 4,49 | 5,69 | 42,07 | 14,02 |
| PUALGS6 | 5,14 | 6,65 | 49,11 | 16,37 |
| PUALGS7 | 5,58 | 6,65 | 62,73 | 20,91 |
| PUALGS8 | 5,77 | 4,77 | 118,12 | 39,37 |

**Tabelle 3: Eingesetze Verhältnisse**

| | Verhältnis NCO-Gruppen zu OH-Gruppen (im Polyol B) | Verhältnis NCO-Gruppen zu OH-Gruppen und COOH/COO^{Θ}-Gruppen (im Alginat und Polyol B) | Verhältnis OH-Gruppen (im Polyol B) zu OH-Gruppen und COOH/COO^{Θ}-Gruppen (im Alginat) | Verhältnis NCO-Gruppen zu COOH/COO^{Θ}-Gruppen (im Alginat) |
|---|---|---|---|---|
| PUALGS1 | 1:1,48 | 1:5,15 | 1:2,48 | 1:1,22 |
| PUALGS2 | 1:1.52 | 1:5,30 | 1:2,48 | 1:1.26 |
| PUALGS3 | 1:1,36 | 1:8,09 | 1:4,96 | 1:2,24 |
| PUALGS4 | 1:1,37 | 1:8,12 | 1:4,95 | 1:2,25 |
| PUALGS5 | 1:1,27 | 1:10,64 | 1:7,40 | 1:3,12 |
| PUALGS6 | 1:1,30 | 1:10,86 | 1:7,38 | 1:3,19 |
| PUALGS7 | 1:1,19 | 1:12,43 | 1:9,44 | 1:3,75 |
| PUALGS8 | 1:0,83 | 1:21,30 | 1:24,76 | 1:6,82 |

Dabei weisen die einzelnen zur Reaktion gebrachten Komponenten die mit Tabelle 2 wiedergegebenen Äquivalente auf. So weist z.B. das eingesetzte Polyol in der Zusammensetzung PUALGS1 7,83 mmol Hydroxylgruppen (12,533 g * 1000 mmol) / 1600 g = 7,83 mmol) auf (vgl. Tabelle 2 - eingesetzte Äquivalente). Dies entspricht der Anzahl an Hydroxylgruppen in dem eingesetzten Polyol (bezogen auf die eingesetzte Masse Polyol). Diese Menge an Hydroxylgruppen ist mit 5,29 mmol Isocyanatgruppen aus dem Isophorondiisocyanat-Prepolymer und 19,43 mmol Hydroxyl-Gruppen und Carboxy- bzw. Carboxylatgruppen aus dem Alginat zur Reaktion gebracht worden, wobei die eingesetzten Äquivalente analog bestimmt werden. Dabei sind die mit Tabelle 3 wiedergegebenen Verhältnisse eingestellt. So beträgt beispielsweise in der Zusammensetzung PUALGS1 das Verhältnis der eingesetzten Anzahl der Isocyanatgruppen (NCO-Gruppen) zur Anzahl der eingesetzten reaktiven Gruppen aus dem Polyol B (OH-Gruppen) und dem Alginat (OH-Gruppen und COOH/ COO^{Θ}-Gruppen) 1 : 5,15 (5,29 : (19,43 + 7,83)). Der erfindungsgemäße Polyurethangelschaum kann somit als untervernetzter Polyurethangelschaum bezeichnet werden.

Zur Herstellung der Polyurethangelschäume wurde in einem ersten Schritt eine Polyol-Polysaccharid-Dispersion, hier eine Polyol-Alginat-Dispersion hergestellt. Hierzu wurde die Polyolkomponente B mit den Hilfs- und Zusatzstoffen in einem Plastikgefäß in der gewünschten Menge vorgelegt und das Alginat in der angegebenen Menge zugegeben. Beide Substanzen wurden in einer offenen Rührapparatur bei 450 U/min 15 min lang vermischt. In einem zweiten Schritt wird diese Polyol-Alginat-Dispersion zu der Isocyanatkomponente zugegeben. Hierzu wurde die Isocyanatkomponente (Komponente A) auf einer oberschaligen Waage, in einem Plastikgefäß, auf 0,01 g genau eingewogen. Im selben Gefäß wurde dann die Polyol-Alginat-Dispersion eingewogen. Diese Mischung wurde mittels eines Laborrührers 45 sek. vermischt. Nach dem Mischvorgang wurde die Masse auf ein Siliconpapier [Separacon 9120-64 weiß, Soell Maria GmbH, Nidda-Eichesldorf - Deutschland] gegossen und mit einem Rakel und einem Erichsen 335 Filmziehgerät [Erichsen GmbH u. Co. KG., Hemer - Deutschland] in 180 µm Schichtdicke ausgestrichen. Direkt anschließend wurde ein hydrophiler absorbierender Polyurethanschaum [Rynel L00562 E, Rynel Inc., Wiscasset, ME, USA laminiert mit Scapa 4005 Dry PU-Foam, Scapa Medical, Luton, UK] auf die hydrophile Seite aufgebracht und mit einer Handwalze angerollt. Die Muster wurden dann für 2 Minuten im Trockenschrank [FDL115, Binder, Tuttlingen, Deutschland] bei 105°C aktiviert. Nach dem Aktivierungsschritt wird das hergestellte Laminat für 72 Std. bei Raumtemperatur gelagert. Aus dem somit hergestellten Laminat werden Wundauflagen ausgestanzt. Die Wundauflagen weisen den mit Figur 2 wiedergegebenen Aufbau auf, mit dem Unterschied, dass die Wundauflagen nicht als Inselverband gefertigt sind und keinen zusätzlichen klebenden Rand aufweisen. Mit Tabelle 4 sind einige Eigenschaften dieser Wundauflagen aufgezeigt.

Hervorzuheben ist hier, dass alle eingesetzten Polyurethangelschäume hervorragende Eigenschaften als Wundkontaktschicht aufweisen. So kann einerseits eine Wundkontaktschicht bereitgestellt werden, die gute bis sehr gute haftklebende Eigenschaften aufweist und andererseits eine Wundkontaktschicht bereitgestellt werden, die eine besonders schnelle Durchtrittsgeschwindigkeit für Wundexsudat bereitstellt.

**Tabelle 4: Eigenschaften der Wundauflagen**

| Eingesetzte Polyurethangelschäume | Klebkraft auf Stahl (Test 1) Mittelwert [N/25 mm] | Anfangsklebrigkeit (Test 2) max. Kraft [N/25 mm] | Durchtrittsgeschwindigkeit (Test 3) [s] |
|---|---|---|---|
| PU-Gel * | | | 110 |
| PUALGS1 | 1,83±0,38 | 2,00±0,38 | 23 |
| PUALGS2 | | | |
| PUALGS3 | 1.38±0,50 | 1,39±0,28 | 30 |
| PUALGS4 | | | |
| PUALGS5 | 1,57±0,71 | 1,22±0,43 | 20 |
| PUALGS6 | | | |
| PUALGS7 | 1,02±0,13 | 1,12±0,38 | 21 |
| PUALGS8 | 0,61±0,01 | 0,87±0,22 | 26 |

| | | | |
|---|---|---|---|
| * nicht anspruchsgemäßes Beispiel mit demselben Aufbau, wobei jedoch als Wundkontaktschicht ein hydrophiles Polyurethan-Elastomer 1 ohne Polysaccharidanteil gemäß EP 1923077 A1 verwendet wurde. | | | |

Zur Untersuchung der Schaumstruktur wurden die oben beschriebenen Polyurethangelschäume auf einen transparenten Polyurethanfilm (PU-Film VP 940-2, Collano Xiro, Buxtehude, Deutschland) aufgebracht. Die Herstellung erfolgte nach dem oben beschriebenen Verfahren, wobei ebenfalls eine Schichtdicke von 180 µm eingestellt wurde.

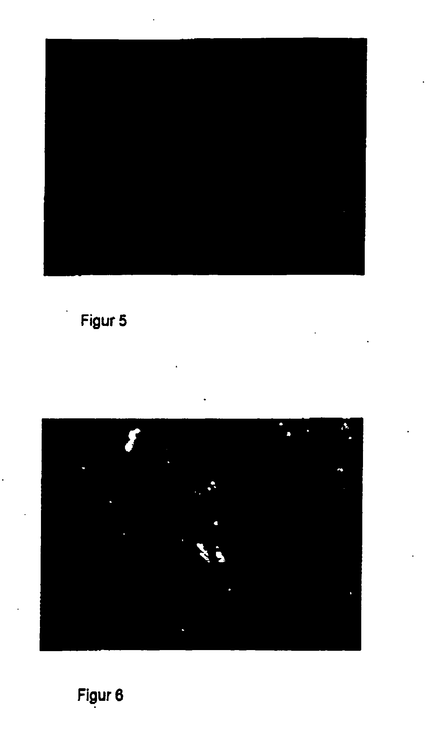

Abbildung 5 zeigt nun eine lichtmikroskopische Aufnahme eines Polyurethangelschaums PUALGS4 mit 9,5 Gew-% Alginatanteil auf einem Polyurethanfilm (PU-Film VP 940-2, Collano Xiro, Buxtehude, Deutschland) in 30-facher Vergrößerung. Es ist deutlich eine geschlossenporige Schaumstruktur zu erkennen. Der Polyurethangelschaum liegt als transparente, homogene Polymermatrix vor. In der Polymermatrix sind keine Alginatpartikel zu erkennen.

Abbildung 6 zeigt eine lichtmikroskopische Aufnahme eines Polyurethangelschaums PUALGS6 mit 13,2-% Alginatanteil auf einem Polyurethanfilm (PU-Film VP 940-2, Collano Xiro, Buxtehude, Deutschland) in 30-facher Vergrößerung. Dieser Polyurethangelschaum weist im Vergleich zu dem mit Abbildung 5 gezeigten Polyurethanschaum einen um ca. 4 Gew.-% höheren Alginatanteil auf. Je höher der Alginatanteil wird desto unregelmäßiger erscheint die Schaumstruktur im Lichtmikroskop.

## Patentansprüche

1. Polyurethangelschaum zur Verwendung als Wundkontaktschicht, **dadurch gekennzeichnet, dass** der Polyurethangelschaum aus mindestens folgenden zur Reaktion gebrachten Komponenten erhalten wird:
a) eine Isocyanatkomponente A mit einer Funktionalität f von f_{A} ≤ 3,
b) eine polymere Polyolkomponente B mit einer Funktionalität f von f_{B} ≤ 6 und
c) ein Polysaccharid C, das mindestens eine Uronsäure oder ein Salz hiervon umfasst,
wobei das Verhältnis der Anzahl der Isocyanatgruppen der Isocyanatkomponente A zur Gesamtzahl der Hydroxylgruppen, Carboxygruppen und Carboxylatgruppen in der polymeren Polyolkomponente B und in dem Polysaccharid C dem Verhältnis 1 : 2 bis 1 : 30 entspricht.

2. Polyurethangelschaum nach Anspruch 1, **dadurch gekennzeichnet, dass** der Polyurethanschaum ein haftklebender Polyurethangelschaum ist.

3. Polyurethangelschaum nach Anspruch 1, **dadurch gekennzeichnet, dass** das Verhältnis der Anzahl der Isocyanatgruppen der Isocyanatkomponente A zur Gesamtzahl der Carboxygruppen und Carboxylatgruppen in dem Polysaccharid C dem Verhältnis 1 : 0,5 bis 1:10 entspricht.

4. Polyurethangelschaum nach mindestens einem der vorangehenden Ansprüche, **dadurch gekennzeichnet, dass** die Isocyanatkomponente A ausgewählt wird aus der Gruppe der aliphatischen Di- oder Polyisocyanate, alicyclischen Di- oder Polyisocyanate oder Mischungen hiervon.

5. Polyurethangelschaum nach mindestens einem der vorangehenden Ansprüche, **dadurch gekennzeichnet, dass** die Isocyanatkomponente A Isophorondiisocyanat, ein Isophorondiisocyanat-Prepolymer oder eine Mischungen hiervon ist, wobei das Isophorondiisocyanat-Prepolymer erhältlich ist aus Isophorondiisocyanat und einem Polyetherpolyol D mit einer Funktionalität f von f_{D} ≤ 3.

6. Polyurethangelschaum. nach mindestens einem der vorangehenden Ansprüche, **dadurch gekennzeichnet, dass** das Polyetherpolyol D ausgewählt wird aus der Gruppe der Polyethylenglykole, Polypropylenglykole, Polyethylen-Polypropylenglykole oder Mischungen hiervon, wobei das Polyetherpolyol D eine mittlere zahlengemittelte molare Masse Mₙ von mindestens Mₙ (D) = 2000 g/ mol und höchstens Mₙ (D) =10.000 g/ mol aufweist.

7. Polyurethangelschaum nach mindestens einem der vorangehenden Ansprüche, **dadurch gekennzeichnet, dass** die Polyolkomponente B ein Polyetherpolyol ist, das eine Funktionalität f von 3 ≤ f_{B} ≤ 6 aufweist.

8. Polyurethangelschaum nach mindestens einem der vorangehenden Ansprüche, **dadurch gekennzeichnet, dass** die Polyolkomponente B ein Polyetherpolyol ist, das eine mittlere zahlengemittelte molare Masse Mₙ von 3.000 bis 10.OD0 g/ mol aufweist.

9. Polyurethangelschaum nach mindestens einem der vorangehenden Ansprüche, **dadurch gekennzeichnet, dass** das Polysaccharid C als Uronsäure Guluronsäure oder ein Salz hiervon, Mannuronsäure oder ein Salz hiervon, Galacturonsäure oder ein Salz hiervon, Glucuronsäure oder ein Salz hiervon, Iduronsäure oder ein Salz hiervon, oder Mischungen dieser Säuren oder deren Salze umfasst.

10. Polyurethangelschaum nach mindestens einem der vorangehenden Ansprüche, **dadurch gekennzeichnet, dass** das Polysaccharid C ausgewählt wird aus der Gruppe Alginsäure oder deren Salze, Hyaluronsäure oder deren Salze, Glucosaminoglycane oder deren Salze, Xanthane oder deren Salze, oder Mischungen dieser Polysaccharide oder deren Salze.

11. Polyurethangelschaum nach mindestens einem der vorangehenden Ansprüche, **dadurch gekennzeichnet, dass** das Polysaccharid C ein Calziumalginat, ein Natriumalginat, ein Natrium-Calziumalginat oder eine Mischungen hiervon ist.

12. Wundauflage (10, 20, 30) umfassend einen Polyurethangelschaum gemäß mindestens einem der Ansprüche 1 bis 11 als Wundkontaktschicht (12, 22, 32).

13. Wundauflage (20, 30) nach Anspruch 12, **dadurch gekennzeichnet, dass** die Wundauflage als absorbierende Schicht (24, 34) einen absorbierenden hydrophilen Polymerschaum oder ein absorbierendes Textilmaterial umfasst.

14. Wundauflage (20, 30) nach Anspruch 12 oder 13, **dadurch gekennzeichnet, dass** die Wundauflage eine absorbierende Schicht (24, 34) umfasst, die mit dem Polyurethangelschaum in direktem Kontakt steht.

## Claims

1. Polyurethane gel foam for use as a wound contact layer, **characterized in that** the polyurethane gel foam is obtained from at least the following components having been made to react:
a) an isocyanate component A having a functionality f of f_{A} ≤ 3,
b) a polymeric polyol component B having a functionality f of f_{B} ≤ 6, and
c) a polysaccharide C comprising at least one uronic acid or salt thereof,
wherein the ratio of the number of isocyanate groups of isocyanate component A to the total number of hydroxyl groups, carboxy groups and carboxylate groups in the polymeric polyol component B and in the polysaccharide C corresponds to a ratio in the range from 1:2 to 1:30.

2. Polyurethane gel foam according to Claim 1, **characterized in that** the polyurethane foam is a pressure-sensitive adhesive polyurethane gel foam.

3. Polyurethane gel foam according to Claim 1, **characterized in that** the ratio of the number of isocyanate groups of isocyanate component A to the total number of carboxy groups and carboxylate groups in the polysaccharide C corresponds to a ratio in the range from 1:0.5 to 1:10.

4. Polyurethane gel foam according to at least one of the preceding claims, **characterized in that** the isocyanate component A is selected from the group of aliphatic di- or polyisocyanates, alicyclic di- or polyisocyanates or mixtures thereof.

5. Polyurethane gel foam according to at least one of the preceding claims, **characterized in that** the isocyanate component A is isophorone diisocyanate, an isophorone diisocyanate prepolymer or a mixture thereof, wherein the isophorone diisocyanate prepolymer is obtainable from isophorone diisocyanate and a polyether polyol D having a functionality f of f_{D} ≤ 3.

6. Polyurethane gel foam according to at least one of the preceding claims, **characterized in that** the polyether polyol D is selected from the group of polyethylene glycols, polypropylene glycols, polyethylene-polypropylene glycols or mixtures thereof, wherein the polyether polyol D has a mean number-average molar mass Mₙ of at least Mₙ (D) = 2000 g/mol and at most Mₙ (D) = 10 000 g/mol.

7. Polyurethane gel foam according to at least one of the preceding claims, **characterized in that** the polyol component B is a polyether polyol that has a functionality f of 3 ≤ f_{B} ≤ 6.

8. Polyurethane gel foam according to at least one of the preceding claims, **characterized in that** the polyol component B is a polyether polyol that has a mean number-average molar mass Mₙ of 3000 to 10 000 g/mol.

9. Polyurethane gel foam according to at least one of the preceding claims, **characterized in that** the polysaccharide C comprises by way of uronic acid guluronic acid or a salt thereof, mannuronic acid or a salt thereof, galacturonic acid or a salt thereof, glucuronic acid or a salt thereof, iduronic acid or a salt thereof, or mixtures of these acids or of their salts.

10. Polyurethane gel foam according to at least one of the preceding claims, **characterized in that** the polysaccharide C is selected from the group alginic acid or salts thereof, hyaluronic acid or salts thereof, glucosaminoglycans or salts thereof, xanthans or salts thereof, or mixtures of these polysaccharides or of their salts.

11. Polyurethane gel foam according to at least one of the preceding claims, **characterized in that** the polysaccharide C is a calcium alginate, a sodium alginate, a sodium calcium alginate or a mixture thereof.

12. Wound dressing (10, 20, 30) comprising a polyurethane gel foam according to at least one of Claims 1 to 11 as a wound contact layer (12, 22, 32).

13. Wound dressing (20, 30) according to Claim 12, **characterized in that** the wound dressing comprises by way of an absorbent layer (24, 34) an absorbent hydrophilic polymeric foam or an absorbent textile material.

14. Wound dressing (20, 30) according to Claim 12 or 13, **characterized in that** the wound dressing comprises an absorbent layer (24, 34) which is in direct contact with the polyurethane gel foam.

## Revendications

1. Mousse de gel de polyuréthane destinée à une utilisation comme couche de contact avec une plaie, **caractérisée en ce que** la mousse de gel de polyuréthane est obtenue à partir d'au moins les composants suivants amenés à réagir :
a) un composant isocyanate A d'une fonctionnalité f de f_{A} ≤ 3,
b) un composant polyol polymère B d'une fonctionnalité f de f_{B} ≤ 6 et
c) un polysaccharide C qui comprend au moins un acide uronique ou un sel de celui-ci,
le rapport du nombre de groupes isocyanate du composant isocyanate A au nombre total de groupes hydroxyle, de groupes carboxy et de groupes carboxylate dans le composant polyol polymère B et dans le polysaccharide C correspondant au rapport 1:2 à 1:30.

2. Mousse de gel de polyuréthane selon la revendication 1, **caractérisée en ce que** la mousse de polyuréthane est une mousse de gel de polyuréthane autoadhésive.

3. Mousse de gel de polyuréthane selon la revendication 1, **caractérisée en ce que** le rapport du nombre de groupes isocyanate du composant isocyanate A au nombre total de groupes carboxy et de groupes carboxylate dans le polysaccharide C correspond au rapport 1:0,5 à 1:10.

4. Mousse de gel de polyuréthane selon au moins l'une quelconque des revendications précédentes, **caractérisée en ce que** le composant isocyanate A est choisi dans le groupe formé par les diisocyanates ou les polyisocyanates aliphatiques, les diisocyanates ou les polyisocyanates alicycliques ou leurs mélanges.

5. Mousse de gel de polyuréthane selon au moins l'une quelconque des revendications précédentes, **caractérisée en ce que** le composant isocyanate A est l'isophoronediisocyanate, un prépolymère d'isophoronediisocyanate ou un mélange de ceux-ci, le prépolymère d'isophoronediisocyanate pouvant être obtenu à partir d'isophoronediisocyanate et d'un polyétherpolyol D d'une fonctionnalité f de f_{D} ≤ 3.

6. Mousse de gel de polyuréthane selon au moins l'une quelconque des revendications précédentes, **caractérisée en ce que** le polyétherpolyol D est choisi dans le groupe formé par les polyéthylèneglycols, les polypropylèneglycols, les polyéthylène-polypropylèneglycols ou leurs mélanges, le polyétherpolyol D présentant une masse molaire numérique moyenne Mₙ d'au moins Mₙ (D) = 2000 g/mole et d'au plus Mₙ (D) = 10 000 g/ mole.

7. Mousse de gel de polyuréthane selon au moins l'une quelconque des revendications précédentes, **caractérisée en ce que** le composant polyol B est un polyétherpolyol, qui présente une fonctionnalité f de 3 ≤ f_{B} ≤ 6.

8. Mousse de gel de polyuréthane selon au moins l'une quelconque des revendications précédentes, **caractérisée en ce que** le composant polyol B est un polyétherpolyol, qui présente une masse molaire numérique moyenne Mₙ de 3000 à 10 000 g/mole.

9. Mousse de gel de polyuréthane selon au moins l'une quelconque des revendications précédentes, **caractérisée en ce que** le polysaccharide C comprend, comme acide uronique, l'acide guluronique ou un sel de celui-ci, l'acide mannuronique ou un sel de celui-ci, l'acide galacturonique ou un sel de celui-ci, l'acide glucuronique ou un sel de celui-ci, l'acide iduronique ou un sel de celui-ci ou les mélanges de ces acides ou de leurs sels.

10. Mousse de gel de polyuréthane selon au moins l'une quelconque des revendications précédentes, **caractérisée en ce que** le polysaccharide C est choisi dans le groupe formé par l'acide alginique ou ses sels, l'acide hyaluronique ou ses sels, les glucosaminoglycanes ou leurs sels, les xanthanes ou leurs sels ou les mélanges de ces polysaccharides ou de leurs sels.

11. Mousse de gel de polyuréthane selon au moins l'une quelconque des revendications précédentes, **caractérisée en ce que** le polysaccharide C est un alginate de calcium, un alginate de sodium, un alginate de sodium-calcium ou un mélange de ceux-ci.

12. Pansement (10, 20, 30) comprenant une mousse de gel de polyuréthane selon au moins l'une quelconque des revendications 1 à 11 comme couche de contact avec une plaie (12, 22, 32).

13. Pansement (20, 30) selon la revendication 12, **caractérisé en ce que** le pansement comprend comme couche absorbante (24, 34) une mousse de polymère hydrophile absorbante ou un matériau textile absorbant.

14. Pansement (20, 30) selon la revendication 12 ou 13, **caractérisé en ce que** le pansement comprend une couche absorbante (24, 34) qui est en contact direct avec la mousse de gel de polyuréthane.
